# EUROPEAN PATENT APPLICATION

(11) **EP 4 831 275 A1**
(43) Date of publication of application: **28.10.2026**
(21) Application number: 25757839.3
(22) Date of filing: 21.02.2025
(51) Int. Cl.: C12N 1/20, C12N 15/10, C12N 15/11, A61K 8/99, A61K 8/60, A61Q 19/00, C12R 1/225

(54) **PDRN, AND PREPARATION METHOD THEREFOR AND USE THEREOF**

(30) Priority: 22.02.2024 CN 202410195149; 22.02.2024 CN 202410195108
(71) Applicant: Bloomage Biotechnology Corporation Limited, Jinan, Shandong 250101 (CN)
(72) Inventor: ZHANG, Weiping, Wuxi, Jiangsu 214122 (CN); LU, Zhen, Jinan, Shandong 250101 (CN); ZHANG, Chen, Wuxi, Jiangsu 214122 (CN); WEN, Liangliang, Jinan, Shandong 250101 (CN); WANG, Ruiyan, Jinan, Shandong 250101 (CN)
(74) Representative: Huang, Liwei
(86) International application number: PCT/CN2025/078618
(87) International publication number: WO 2025/176205

(57) **Abstract**

The present invention provides a polydeoxyribonucleotide/polyribonucleotide (PDRN) and a preparation method therefor as well as use thereof, belonging to the field of biotechnology. The PDRN has the GC content of 39%-42%. The GC% in gDNA in PDRN prepared by using the method recited in the present invention has a similarity of 98% or more to the GC% of human and is closer to the GC% of a human body compared with that in salmon-derived PDRN, with higher safety and efficacy. Furthermore, compared with the extraction of PDRN from semen of salmon in the prior art, the preparation method of PDRN disclosed in the present invention is simpler and more convenient.

## Description

### CROSS-REFERENCE TO RELATED APPLICATION

This application claims the priority of Chinese Patent Application No. 202410195149.3 filed with the China National Intellectual Property Administration on February 22, 2024 and entitled "PDRN AND PREPARATION METHOD THEREFOR AS WELL AS USE THEREOF", the disclosure of which is incorporated by reference herein in its entirety as part of the present invention.

This application claims the priority of Chinese Patent Application No. 202410195108.4 filed with the China National Intellectual Property Administration on February 22, 2024 and entitled "PREPARATION METHOD OF PDRN USING *Lactobacillus sakei* AND APPLICATION THEREOF", the disclosure of which is incorporated by reference herein in its entirety as part of the present invention.

### TECHNICAL FIELD

The present invention relates to the field of biotechnology, particularly to a PDRN and a preparation method therefor as well as use thereof.

### BACKGROUND

PDRN (polydeoxyribonucleotide/polyribonucleotide) is a biomacromolecule substance derived from salmon testis cells, which consists of multiple base pairs linked via phosphodiester bonds. Essentially, it is a mixture composed of salmon genetic materials with different fragment sizes. As early as 1952, Italy's Mastelli Company extracted PDRN fragments of varying sizes from salmon testis cells, inspired by fishermen's practice of using salmon testes to treat wounds. The company also conducted research on its pharmacological activity and other related properties. Experimental results show that nucleic acid fragments (PDRN) derived from male salmon testis cells can significantly promote human cell regeneration, accelerate wound healing and reduce scar formation. Subsequent continuous studies have further revealed that PDRN also possesses anti-inflammatory, tissue-repairing, pro-angiogenic and anti-ischemic effects, as well as efficacy in improving diabetic foot conditions.

In most modern industries, PDRN raw materials are extracted from testis cells of deep-sea salmon. For example, METHOD FOR ISOLATING POLYDEOXYRIBONUCLEOTIDES FROM FISH SEMEN disclosed in CN107287186A, PREPARATION AND USE OF SMALL-MOLECULE POLYDEOXYRIBONUCLEOTIDE disclosed in CN110747194A, EFFICIENT PREPARATION METHOD AND USE OF TOPICAL PDRN disclosed in CN112315836A, PREPARATION METHOD AND USE OF POLYDEOXYRIBONUCLEOTIDE disclosed in CN115074357A, etc. Although the above-mentioned processes have been the main ways to acquire PDRN raw materials, the extraction of PDRN from salmon testis cells can barely satisfy the surging demands for PDRN raw materials nowadays with the continuous depletion of marine fishery resources. In addition, fuel spills and nuclear wastewater discharge from numerous large vessels plying ocean routes as well as domestic waste disposal in coastal areas have severely damaged the marine environment. The consequent heavy metal accumulation, chromosome aberration, gene mutation and others in marine organisms can severely affect the safety of PDRN raw materials derived from salmon testis cells.

In summary, it is urgent to develop diverse production routes for PDRN to address the problems of single source and poor safety.

### SUMMARY

The present invention is the first to prepare PDRN from a variety of species including microorganisms, algae and animals, which resolves the poor safety of PDRN caused by marine environmental pollution while diversifying the sources of PDRN products, thereby facilitating better quality control of raw materials for PDRN extraction.

In one aspect, the present invention provides a strain of *Lactobacillus sakei*, wherein the *Lactobacillus sakei* is *Lactobacillus sake* LS-01 which is deposited at the China Center for Type Culture Collection under the deposit number CCTCC NO: M 20232285.

In the present invention, a strain of *Lactobacillus sakei* with high activity and suitable for preparing PDRN is screened, i.e., *Lactobacillus sake* LS-01. Its 16S rDNA sequence is as shown in SEQ ID NO. 1. As early as 2014, *Lactobacillus sakei* was approved by the National Health Commission and included in the List of New Food Raw Materials as an edible microbial strain. The present invention is the first to utilize *Lactobacillus sakei* for PDRN production, providing a new raw material and a novel approach for PDRN production.

In another aspect, the present invention further provides a microbial agent comprising the *Lactobacillus sakei*.

Optionally, the *Lactobacillus sakei* is *Lactobacillus sake* LS-01 which is deposited at the China Center for Type Culture Collection under the deposit number CCTCC NO: M 20232285.

Optionally, the microbial agent contains the dried bacterial cells and/or wet bacterial cells of the above-mentioned *Lactobacillus sakei* with a viable cell count of ≥10⁶ CFU/g.

The microbial agent can comprise various forms of *Lactobacillus sakei* preparations, such as a live bacterial suspension of *Lactobacillus sakei*, an inactivated bacterial suspension of *Lactobacillus sakei*, metabolites of *Lactobacillus sakei* and an extract of *Lactobacillus sakei*.

Those skilled in the art can culture the *Lactobacillus sakei* of the present invention according to the general culture method of the *Lactobacillus sakei*.

In another aspect, the present invention further provides a strain of *Bacillus pumilus*, wherein the *Bacillus pumilus* is *Bacillus pumilus* Bp-1 which is deposited at the China Center for Type Culture Collection under the deposit number CCTCC NO: M 2025198.

In another aspect, the present invention further provides a strain of *Bacillus pumilus*, wherein the *Bacillus pumilus* is *Bacillus pumilus* Bp-2 which is deposited at the China Center for Type Culture Collection under the deposit number CCTCC NO: M 2025199.

In the present invention, a strain of *Bacillus pumilus* with high activity and suitable for preparing PDRN is screened.

In another aspect, the present invention further provides a microbial agent comprising the *Bacillus pumilus*.

Those skilled in the art can culture the *Bacillus pumilus* of the present invention according to the general culture method of the *Bacillus pumilus*.

In another aspect, the present invention further provides a strain of *Komagataella sp.*, wherein the *Komagataella sp.* is *Komagataella sp.* GS115 which is deposited at the China Center for Type Culture Collection under the deposit number CCTCC NO: M 2025200.

In the present invention, a strain of *Komagataella sp.* with high activity and suitable for preparing PDRN is screened.

In another aspect, the present invention further provides a microbial agent comprising the *Komagataella sp.*

Those skilled in the art can culture the *Komagataella sp.* of the present invention according to the general culture method of the *Komagataella sp.*

The *Komagataella sp.* was formerly designated as *Pichia pastoris*.

In another aspect, the present invention further provides a preparation method of PDRN, the method comprising the following steps:
Step 1, collecting materials, and then lysing the materials to prepare a lysate;
Step 2, salting out the lysate to prepare a salting-out supernatant;
Step 3, adding an organic solvent to the salting-out supernatant for precipitation to prepare a crude product; and
Step 4, drying the crude product to obtain a product.

Optionally, the materials comprise microorganisms, animals and/or algae;
more optionally, the animals comprise one or more selected from *Cavia porcellus*, *Caenorhabditis japonica*, *Drosophila ananassae*, *Branchiostoma floridae*, *Oryzias latipes* and *Gallus gallus*, and more optionally the animals comprise *Cavia porcellus*;
Optionally, the algae comprise freshwater algae, more optionally, the algae comprise *Anabaena variabilis*;
More optionally, the microorganisms comprise one or more selected from *Lactobacillus, Bacillus, Eubacterium* and yeasts, and more optionally the microorganisms comprise one or more selected from *Lactobacillus sakei*, *Eubacterium rectale*, *Bacillus pumilus*, *Komagataella sp.* and *Lactobacillus reuteri.*

Further, the method comprises the following steps:
Step 1, collecting materials, resuspending the materials, and then adding an enzyme for lysis to prepare a lysate;
Step 2, adding a salt and a protein denaturant to the lysate for salting out to prepare a salting-out supernatant;
Step 3, adding an organic solvent to the salting-out supernatant for precipitation to prepare a crude product; and
Step 4, drying the crude product to obtain a product.

Optionally, the enzyme comprises one or more selected from Labiase derived from *Streptomyces fulvissimus*, mutanolysin, lysostaphin, achromopeptidase, papain, Proteinase K and snailase; optionally, the final concentration of the enzyme added is 0.2-2 mg/mL.

The enzyme comprises any enzyme capable of destroying Gram-positive bacterial cell walls or yeast cell walls.

Optionally, the concentration of the enzyme added may be 0.2 mg/mL, 0.3 mg/mL, 0.4 mg/mL, 0.5 mg/mL, 0.6 mg/mL, 0.7 mg/mL, 0.8 mg/mL, 0.9 mg/mL, 1 mg/mL, 2 mg/mL and any value therebetween.

Optionally, the lysis step in the Step 1 comprises: upon addition of the enzyme, performing constant-temperature incubation at 25°C-60°C for 1-5 h.

The temperature for constant-temperature incubation may be selected from 25°C, 26°C, 27°C, 28°C, 29°C, 30°C, 31°C, 32°C, 33°C, 34°C, 35°C, 36°C, 37°C, 38°C, 39°C, 40°C, 50°C, 55°C, 56°C, 60°C and any value therebetween.

The time for constant-temperature incubation can be selected from any value of 1 h, 2 h, 3 h, 4 h and 5 h.

Optionally, the salt comprises one or more selected from sodium chloride, potassium acetate, sodium acetate, ammonium sulfate and sodium sulfate; optionally, the salt added is sodium chloride, and optionally, the final concentration of the salt added is 1-5 M.

The final concentration of the salt may be 1 M, 1.5 M, 2 M, 2.5 M, 3 M, 3.5 M, 4 M, 4.5 M, 5 M and any value therebetween.

Optionally, the protein denaturant comprises one or more selected from guanidine hydrochloride, phenol and surfactants, wherein the surfactants comprise one or more selected from sodium dodecyl sulfate, sodium lauroyl sarcosinate, octylphenol ethoxylate, polyoxyethylene sorbitan monolaurate, polyoxyethylene sorbitan monopalmitate, polyoxyethylene sorbitan monostearate, polyoxyethylene sorbitan tristearate and polyoxyethylene sorbitan monooleate, optionally, the protein denaturant is sodium dodecyl sulfate; based on the mass percentage of the cell lysate, the concentration of the protein denaturant added is 0.5%-2%.

Based on the mass percentage of the cell lysate, the concentration of the protein denaturant added may be 0.5%, 0.6%, 0.7%, 0.8%, 0.9%, 1%, 1.1%, 1.2%, 1.3%, 1.4%, 1.5%, 1.6%, 1.7%, 1.8%, 1.9%, 2.0% and any value therebetween.

It can be understood that those skilled in the art may select known protein denaturants for this treatment step, provided that the salting out of the final product can be achieved. Accordingly, no specific methods or equipment are limited herein.

Optionally, the condition of the salting out comprises: incubating at 40°C-80°C for 1-3 h.

The temperature for constant-temperature incubation can be selected from 40°C, 45°C, 50°C, 55°C, 60°C, 65°C, 70°C, 75°C, 80°C and any value therebetween.

The time for constant-temperature incubation can be selected from 1 h, 1.5 h, 2 h, 2.5 h, 3 h and any value therebetween.

Optionally, the organic solvent comprises one or more selected from ethanol, methanol and isopropanol.

Optionally, the organic solvent is ethanol; more optionally, absolute ethanol.

Optionally, the mass ratio of the salting-out supernatant to the organic solvent is 1: (1-5); more optionally, 1:2.5.

The mass ratio of the salting-out supernatant to the organic solvent can be selected from 1:1, 1:1.1, 1:1.2, 1:1.3, 1:1.4, 1:1.5, 1:1.6, 1:1.7, 1:1.8, 1:1.9, 1:2, 1:2.1, 1:2.2, 1:2.3, 1:2.4, 1:2.5, 1:2.6, 1:2.7, 1:2.8, 1:2.9, 1:3, 1:3.1, 1:3.2, 1:3.3, 1:3.4, 1:3.5, 1:3.6, 1:3.7, 1:3.8, 1:3.9, 1:4, 1:4.1, 1:4.2, 1:4.3, 1:4.4, 1:4.5, 1:4.6, 1:4.7, 1:4.8, 1:4.9, 1:5 and any value therebetween.

Optionally, the precipitation time is 20-60 min; more optionally, 30 min.

The precipitation time can be selected from 20 min, 21 min, 22 min, 23 min, 24 min, 25 min, 26 min, 27 min, 28 min, 29 min, 30 min, 31 min, 32 min, 33 min, 34 min, 35 min, 36 min, 37min, 38 min, 39 min, 40 min, 41 min, 42min, 43 min, 44min, 45 min, 46 min, 47 min, 48 min, 49 min, 50 min, 51 min, 52 min, 53 min, 54 min, 55 min, 56 min, 57 min, 58 min, 59 min, 60 min and any value therebetween.

Optionally, in the Step 3, sodium acetate is added to the salting-out supernatant; more optionally, the concentration of the sodium acetate is 0.1-0.5 M; more optionally, 0.3 M.

The concentration of the sodium acetate can be selected from 0.1 M, 0.2 M, 0.3 M, 0.4 M, 0.5 M and any value therebetween.

Optionally, in the Step 3, a step of rinsing and centrifugation is included. The supernatant after centrifugation is discarded to obtain the crude product.

Optionally, in the Step 4, the drying comprises the steps of dehydration and vacuum drying.

It can be understood that those skilled in the art may adopt known drying techniques for this treatment step, so long as dehydration and drying of the final product are accomplished. Therefore, no specific procedures are defined herein.

Optionally, the materials comprise microorganisms, animals and/or algae;
more optionally, the animals comprise one or more selected from *Cavia porcellus*, *Caenorhabditis japonica*, *Drosophila ananassae*, *Branchiostoma floridae*, *Oryzias latipes* and *Gallus gallus*, and more optionally the animals comprise *Cavia porcellus*;
more optionally, the algae comprise freshwater algae, and more optionally the algae comprise *Anabaena variabilis*;
more optionally, the microorganisms comprise one or more selected from *Lactobacillus, Bacillus, Eubacterium* and yeasts, and more optionally, the microorganisms comprise one or more selected from *Lactobacillus sakei*, *Eubacterium rectale*, *Bacillus pumilus*, *Komagataella sp*. and *Lactobacillus reuteri*.

The *Lactobacillus sakei* is *Lactobacillus sake* LS-01 and/or *Lactobacillus sakei* CICC 21858.

In an optional embodiment, the *Lactobacillus sakei* is *Lactobacillus sake* LS-01 which is deposited at the China Center for Type Culture Collection (CCTCC) under the deposit number CCTCC NO: M 20232285.

In an optional embodiment, the *Lactobacillus sakei* CICC 21858 has the deposit number CICC 21858 and is purchased from the China Center of Industrial Culture Collection.

In an optional embodiment, the *Lactobacillus rhamnosus* has the deposit number CGMCC 1.8882 and is purchased from the Institute of Microbiology, Chinese Academy of Sciences.

The *Eubacterium rectale* is *Eubacterium rectale* ATCC 33656 and/or *Eubacterium rectale* BPB22.

In an optional embodiment, the *Eubacterium rectale* ATCC 33656 has the deposit number ATCC 33656 and is purchased from the American Type Culture Collection.

In an optional embodiment, the *Eubacterium rectale* BPB22 has the deposit number CCTCC NO: M 20232177 and is purchased from the China Center for Type Culture Collection.

The *Bacillus pumilus* is *Bacillus pumilus* Bp-1, *Bacillus pumilus* Bp-2 and/or *Bacillus pumilus* ATCC 21143.

In an optional embodiment, the *Bacillus pumilus* Bp-1 is deposited at the China Center for Type Culture Collection under the deposit number CCTCC NO: M 2025198.

In an optional embodiment, the *Bacillus pumilus* Bp-2 is deposited at the China Center for Type Culture Collection under the deposit number CCTCC NO: M 2025199.

In an optional embodiment, the *Bacillus pumilus* ATCC 21143 is deposited at the American Type Culture Collection under the deposit number ATCC No. 21143.

The *Komagataella sp.* is *Komagataella sp.* GS115 and/or *Komagataella sp.* CICC 33158;
The *Komagataella sp.* was formerly designated as *Pichia pastoris*.

In an optional embodiment, the *Komagataella sp.* GS115 is deposited at the China Center for Type Culture Collection (CCTCC) under the deposit number CCTCC NO: M 2025200.

In an optional embodiment, the *Komagataella sp.* CICC 33158 is deposited at the China Center of Industrial Culture Collection under the deposit number CICC 33158.

Those skilled in the art can understand that the above-mentioned strains can be cultured by the conventional method to obtain bacterial and fungal cells, and no further restrictions are imposed herein.

In an optional embodiment, the guinea pigs described herein are purchased from Liaoning Changsheng Biotechnology Co., Ltd., and are of conventional grade.

In an optional embodiment, the *Anabaena variabilis* is deposited at the American Type Culture Collection under the deposit number ATCC 29413.

Further, when the materials are microorganisms and/or algae, the step 1 further comprises a step of high-pressure homogenization. The high-pressure homogenization is performed at the pressure of 200-3000 bar with the number of cycles being no less than 1.

Optionally, the pressure for high-pressure homogenization is 800-1200 bar.

Optionally, the number of cycles for high-pressure homogenization is 1-5.

The pressure for a homogenization and lysis process can be selected from 800 bar, 850 bar, 900 bar, 950 bar, 1000 bar, 1050 bar, 1100 bar, 1150 bar, 1200 bar and any value therebetween.

The number of cycles for high-pressure homogenization may be 1, 2, 3, 4, 5 and more, provided that a final product PDRN with a corresponding molecular weight is obtained. Accordingly, no further limitations are imposed herein on the number of cycles and relevant equipment and instruments.

According to the present invention, PDRNs with different molecular weights can be prepared by using filtration or chromatography in step 1 based on actual production demands.

The filtration includes, but is not limited to, pleated filtration, filter paper filtration, bag filtration, microfiltration, ultrafiltration and nanofiltration.

The chromatography includes, but is not limited to, adsorption column chromatography, thin-layer chromatography, polyamide thin-layer chromatography, ion-exchange chromatography, size-exclusion chromatography, affinity chromatography, chromatofocusing, etc.

Further, the filtration can be performed using corresponding membranes such as ultrafiltration membranes, including but not limited to hollow fiber membranes, spiral-wound ultrafiltration membranes, flat sheet membranes and tubular membranes. A suitable filter membrane can be selected according to actual requirements, so no further limitations are set forth herein regarding the types and corresponding parameters of ultrafiltration membranes.

Further, the filtration can be performed using corresponding membranes such as nanofiltration membranes, including but not limited to porous nanofiltration membranes, jet nanofiltration membranes, hollow fiber nanofiltration membranes, multilayer nanofiltration membranes and nanofiltration composite membranes. A suitable filter membrane can be selected according to actual requirements, so no further limitations are set forth herein regarding the types and corresponding parameters of nanofiltration membranes.

Further, the size-exclusion chromatography includes, but is not limited to, agarose-based size-exclusion chromatography, dextran-based size-exclusion chromatography, pumice-based size-exclusion chromatography, agar-based size-exclusion chromatography, agarose-based size-exclusion chromatography, polyvinyl alcohol-based size-exclusion chromatography and polyacrylamide-based size-exclusion chromatography. Suitable molecular sieves can be selected for filtration according to actual requirements, so no further limitations are imposed herein on the types and corresponding parameters of molecular sieves.

The above-mentioned filtration methods are suitable for the preparation of PDRNs with different molecular weights derived from microorganisms, animals, plants and algae. No further limitations are set forth herein.

Further, when the materials are microorganisms or algae, the method comprises the following steps:
Step 1, collecting materials, resuspending the materials and then adding an enzyme for lysis, and subsequently performing high-pressure homogenization to prepare a lysate;
Step 2, adding a salt and a protein denaturant to the lysate for salting out to prepare a salting-out supernatant;
Step 3, adding an organic solvent to the salting-out supernatant for precipitation to prepare a crude product; and
Step 4, dehydrating and drying the crude product to obtain a product.

In an optional embodiment, when the materials are microorganisms, a preparation method of PDRN is provided, the method comprises the following steps:
Step 1, inoculating microbial cells or a microbial agent thereof into a culture medium, and culturing at 25°C-40°C and 100-200 rpm for 1-12 h to obtain a seed solution; inoculating the seed solution into the culture medium in an inoculation volume of 1%-10%, followed by culturing at 25°C-40°C for 10-20 h to obtain a fermentation broth;
Step 2, centrifuging and collecting the microbial cells in the fermentation broth, resuspending the microbial cells with a buffer, and then adding an enzyme for constant-temperature incubation, wherein the final concentration of the enzyme added is 0.2-0.5 mg/mL, and the constant-temperature incubation is performed at 25°C-50°C for 1-5 h; subsequently, performing microbial cell disruption using a homogenization lysis process, wherein the homogenization lysis process is carried out using a high-pressure homogenizer at the pressure of 800-1200 bar for 1-5 passes to prepare a high-pressure homogenate;
Step 3, adding a salt and a surfactant to the prepared high-pressure homogenate for salting-out, wherein the salt is sodium chloride, and the final concentration of the sodium chloride added is 1-5 M; the protein denaturant is sodium dodecyl sulfate, and the final concentration of the sodium dodecyl sulfate added is 0.5%-2%;incubating at 40°C-80°C for 1-3 h after adding the salt and the protein denaturant, followed by centrifuging and collecting a supernatant to prepare a salting-out supernatant;
Step 4, adding absolute ethanol and sodium acetate to the salting-out supernatant, wherein the mass ratio of the salting-out supernatant to absolute ethanol is 1: (1-5), the concentration of the sodium acetate is 0.1-0.5 M, and then precipitating for 20-60 min, rinsing and centrifuging to prepare a crude product; and
Step 5, dehydrating and drying the crude product to obtain a product.

Further, the microorganisms comprise one or more selected from *Lactobacillus, Bacillus, Eubacterium* and yeasts;
Still further, the microorganisms comprise one or more selected from *Lactobacillus sakei*, *Eubacterium rectale*, *Bacillus pumilus*, *Komagataella sp.* and *Lactobacillus reuteri*;
The *Komagataella sp.* was formerly designated as *Pichia pastoris*.

In an optional embodiment, when the materials are algae, a preparation method of PDRN is provided, the method comprising the following steps:
Step 1, inoculating algae into a culture medium, and performing static culture under the illumination conditions of 10°C-50°C and 10-50 µE/(m²·s) for 1-7 days of static culture to obtain a culture solution;
Step 2, centrifuging and collecting the cells in the culture solution, resuspending the cells with a buffer, and then adding an enzyme for constant-temperature incubation, wherein the final concentration of the enzyme added is 0.2-0.5 mg/mL, and the constant-temperature incubation is performed at 25°C-50°C for 1-5 h; subsequently, performing algal cell disruption using a homogenization lysis process, wherein the homogenization lysis process is carried out using a high-pressure homogenizer at the pressure of 800-1200 bar for 1-5 passes to prepare a high-pressure homogenate;
Step 3, adding a salt and a surfactant to the prepared high-pressure homogenate for salting-out, wherein the salt is sodium chloride, and the final concentration of the sodium chloride added is 1-5 M; the protein denaturant is sodium dodecyl sulfate, and the final concentration of the sodium dodecyl sulfate added is 0.5%-2%; incubating at 40°C-80°C for 1-3 h after adding the salt and the protein denaturant, followed by centrifuging and collecting a supernatant to prepare a salting-out supernatant;
Step 4, adding absolute ethanol and sodium acetate to the salting-out supernatant, wherein the mass ratio of the salting-out supernatant to absolute ethanol is 1: (1-5), the concentration of the sodium acetate is 0.1-0.5 M, and then precipitating for 20-60 min, rinsing and centrifuging to prepare a crude product; and
Step 5, dehydrating and drying the crude product to obtain a product.

Further, when the materials are animals, the method comprises the following steps:
Step 1, collecting materials, resuspending the materials, and then adding an enzyme for lysis to prepare a lysate;
Step 2, adding a salt and a protein denaturant to the lysate for salting out to prepare a salting-out supernatant;
Step 3, adding an organic solvent to the salting-out supernatant for precipitation to prepare a crude product; and
Step 4, dehydrating and drying the crude product to obtain a product.

In an optional embodiment, when the materials are animals, a preparation method of PDRN is provided, the method comprising the following steps:
Step 1, collecting animal tissues, and then digesting the collected animal tissues to prepare a tissue lysate;
Step 2, adding a salt and a surfactant to the prepared tissue lysate for salting-out, wherein the salt is sodium chloride, and the final concentration of the sodium chloride added is 1-5 M; the protein denaturant is sodium dodecyl sulfate, and the final concentration of the sodium dodecyl sulfate added is 0.5%-2%;incubating at 40°C-80°C for 1-3 h after adding the salt and the protein denaturant, followed by centrifuging and collecting a supernatant to prepare a salting-out supernatant;
Step 3, adding absolute ethanol and sodium acetate to the salting-out supernatant, wherein the mass ratio of the salting-out supernatant to absolute ethanol is 1: (1-5), the concentration of the sodium acetate is 0.1-0.5 M, and then precipitating for 20-60 min, rinsing and centrifuging to prepare a crude product; and
Step 4, dehydrating and drying the crude product to obtain a product.

Optionally, the animal tissues comprise guinea pig liver tissues, muscle tissues and kidney tissues. The guinea pig tissues are ground into fine powders at low temperature and then resuspended and evenly mixed, and subsequently an enzyme is added for lysis.

In another aspect, the present invention further provides use of microorganisms, animals and/or algae in the preparation of a PDRN product;
Optionally, the animals comprise one or more selected from *Cavia porcellus*, *Caenorhabditis japonica*, *Drosophila ananassae*, *Branchiostoma floridae*, *Oryzias latipes* and *Gallus gallus*, more optionally, the animals comprise *Cavia porcellus*;
Optionally, the algae comprise freshwater algae, more optionally, the algae comprise *Anabaena variabilis*;
Optionally, the microorganisms comprise one or more selected from *Lactobacillus, Bacillus, Eubacterium* and yeasts, and more optionally the microorganisms comprise one or more selected from *Lactobacillus sakei*, *Eubacterium rectale*, *Bacillus pumilus*, *Komagataella sp.* and *Lactobacillus reuteri*.

In another aspect, the present invention further provides use of *Lactobacillus sakei* in the preparation or production of PDRN.

In another aspect, the present invention further provides use of *Eubacterium rectale* in the preparation or production of PDRN.

In another aspect, the present invention further provides use of *Bacillus pumilus* in the preparation or production of PDRN.

In another aspect, the present invention further provides use of *Komagataella sp.* in the preparation or production of PDRN.

In another aspect, the present invention further provides use of *Anabaena variabilis* in the preparation or production of PDRN.

In another aspect, the present invention further provides use of *Cavia porcellus* in the preparation or production of PDRN.

In another aspect, the present invention further provides a PDRN, wherein the GC content of the PDRN is 40.5%-42%.

The GC content of the PDRN prepared by using the method is 40.5%-42%.

Optionally, the GC content of the PDRN may be selected from 40.5%, 40.6%, 40.7%, 40.8%, 40.9%, 41%, 41.1%, 41.2%, 41.3%, 41.4%, 41.5%, 41.6%, 41.7%, 41.8%, 41.9%, 42% and any value therebetween.

Optionally, the GC content of the PDRN may be 40.5%-41.5%.

Further, the weight-average molecular weight of the PDRN is 100 kDa-500 kDa;
and/or, in percentage by mass, the protein content of the PDRN is less than or equal to 0.5%; and/or the nucleic acid content of the PDRN is more than or equal to 96.3%.

Optionally, the weight-average molecular weight of the PDRN can be selected from 100 kDa, 110 kDa, 120 kDa, 130 kDa, 140 kDa, 150 kDa, 160 kDa, 170 kDa, 180 kDa, 190 kDa, 200 kDa, 210 kDa, 220 kDa, 230 kDa, 240 kDa, 250 kDa, 260 kDa, 270 kDa, 280 kDa, 290 kDa, 300 kDa, 310 kDa, 320 kDa, 330 kDa, 340 kDa, 350 kDa, 360 kDa, 370 kDa, 380 kDa, 390 kDa, 400 kDa, 410 kDa, 420 kDa, 430 kDa, 440 kDa, 450 kDa, 460 kDa, 470 kDa, 480 kDa, 490 kDa, 500 kDa and any value therebetween.

Optionally, the protein content of the PDRN is less than or equal to 0.5%, 0.45%, 0.4%, 0.35%, 0.3%, 0.25%, 0.2%, 0.15%, 0.1%, 0.05%, 0.01%, 0% and any value therebetween.

Optionally, the nucleic acid content of the PDRN is more than or equal to 96%, further, the nucleic acid content of the PDRN may be 96%, 96.1%, 96.2%, 96.3%, 96.4%, 96.5%, 96.6%, 96.7%, 96.8%, 96.9%, 97.0%, 97.1%, 97.2%, 97.3%, 97.4%, 97.5%, 97.6%, 97.7%, 97.8%, 97.9%, 98.0%, 98.1%, 98.2%, 98.3%, 98.4%, 98.5%, 98.6%, 98.7%, 98.8%, 98.9%, 99.0%, 99.1%, 99.2%, 99.3%, 99.4%, 99.5%, 99.6%, 99.7%, 99.8%, 99.9%, 100.0% and any value therebetween.

Optionally, the purity (OD₂₆₀/OD₂₈₀) of the PDRN is 1.8-2.0.

The purity (OD₂₆₀/OD₂₈₀) of the PDRN may be 1.8, 1.9, 2.0 and any value therebetween.

Optionally, the PDRN is white or off-white particle or powder.

Optionally, the PDRN has good solubility. 1 g of PDRN product can be completely dissolved in 1 L of water within 30 min.

More optionally, the dissolution time may be 0.5 min, 1 min, 1.5 min, 2 min, 2.5 min, 3 min, 3.5 min, 4 min, 4.5 min, 5 min, 5.5 min, 6 min, 6.5 min, 7 min, 7.5 min, 8 min, 8.5 min, 9min, 9.5 min, 10 min, 10.5 min, 11 min, 11.5 min, 12 min, 12.5 min, 13 min, 13.5 min, 14 min, 14.5 min, 15 min, 15.5 min, 16 min, 16.5 min, 17 min, 17.5 min, 18 min, 18.5 min, 19 min, 19.5 min, 20 min, 20.5 min, 21 min, 21.5 min, 22 min, 22.5 min, 23 min, 23.5 min, 24 min, 24.5 min, 25 min, 25.5 min, 26 min, 26.5 min, 27 min, 27.5 min, 28 min, 28.5 min, 29 min, 29.5 min, 30 min and any value therebetween.

Optionally, the PDRN comprises PDRNs derived from microorganisms, animals and/or algae.

Further, the PDRN comprises PDRN derived from *Lactobacillus sakei*.

Further, the PDRN comprises PDRN derived from *Eubacterium rectale*.

Further, the PDRN comprises PDRN derived from *Bacillus pumilus*.

Further, the PDRN comprises PDRN derived from *Komagataella sp*.

Further, the PDRN comprises PDRN derived from *Lactobacillus reuteri*.

Further, the PDRN comprises PDRN derived from *Anabaena variabilis*.

Further, the PDRN comprises PDRN derived from guinea pigs.

In another aspect, the present invention further provides a composition, the composition comprising the PDRN.

Optionally, the PDRN comprises PDRNs derived from microorganisms, animals and/or algae.

Further, the PDRN comprises PDRN derived from *Lactobacillus sakei*.

Further, the PDRN comprises PDRN derived from *Bacillus pumilus*.

Further, the PDRN comprises PDRN derived from *Eubacterium rectale*.

Further, the PDRN comprises PDRN derived from *Komagataella sp.*

Further, the PDRN comprises PDRN derived from *Lactobacillus reuteri*.

Further, the PDRN comprises PDRN derived from *Anabaena variabilis*.

Further, the PDRN comprises PDRN derived from guinea pigs.

Still further, the composition further comprises excipients.

The excipients may be suitable solvents, propellants, solubilizers, cosolvents, emulsifiers, coloring agents, binders, disintegrants, fillers, lubricants, wetting agents, osmotic pressure regulators, stabilizers, glidants, flavoring agents, preservatives, suspending agents, coating materials, aromatic agents, antiadherents, chelating agents, penetration enhancers, pH regulators, buffering agents, plasticizers, surfactants, foaming agents, defoamers, thickeners, inclusion agents, humectants, absorbents, diluents, flocculants, deflocculants, filter aids, release retardant, etc.

The composition of the present invention can be prepared by using conventional methods. In the composition, one or more diluents or carriers can be added, such as include aqueous solutions, pills, tablets, capsules, granules, powders, troches, syrups, emulsions and suspensions.

The composition of the present invention can be prepared into any preparations conventionally prepared in the art. For example, it can be prepared into creams, lotions, facial masks, foundations, medical devices, hair cosmetics and other preparations. Further, examples include skin lotion, skin softener, hydrafacial injection, skin toner, astringent, cosmetic lotion, lotion, moisturizing lotion, nourishing lotion, massage cream, nourishing cream, moisturizing cream, hand cream, foundation, essence, nourishing essence, facial mask, soap, cleansing foam, cleansing milk, cleansing cream, body lotion or body wash.

In the composition of the present invention, in addition to PDRN as an essential component, other ingredients conventionally formulated in cosmetics may be incorporated as needed. Examples include oily components, humectants, surfactants, organic pigments, inorganic pigments, ultraviolet absorbers, preservatives, antimicrobial agents, antioxidants, plant extracts, pH regulators, alcohols, colorants, fragrances, blood circulation promoters, cooling agents, antiperspirants, or purified water.

In another aspect, the present invention further provides use of the algae, the microorganisms or microbial agents thereof or the PDRN or the composition in the preparation of products for promoting cell proliferation, cell migration and collagen generation and products for exerting anti-inflammatory and soothing effects and/or repairing damaged skin barriers.

The algae comprise freshwater algae, more optionally, the algae comprise *Anabaena variabilis*.

The microorganisms comprise one or more selected from *Lactobacillus, Bacillus, Eubacterium* and yeasts, and more optionally the microorganisms comprise one or more selected from *Lactobacillus sakei*, *Eubacterium rectale*, *Bacillus pumilus*, *Komagataella sp.* and *Lactobacillus reuteri*.

Optionally, the PDRN comprises PDRNs derived from microorganisms, animals and/or algae.

Further, the PDRN comprises PDRN derived from *Lactobacillus sakei*.

Further, the PDRN comprises PDRN derived from *Bacillus pumilus*.

Further, the PDRN comprises PDRN derived from *Eubacterium rectale*.

Further, the PDRN comprises PDRN derived from *Komagataella sp*.

Further, the PDRN comprises PDRN derived from *Lactobacillus reuteri*.

Further, the PDRN comprises PDRN derived from *Anabaena variabilis*.

Further, the PDRN comprises PDRN derived from guinea pigs.

The collagen is Pro-collagen type I α-1.

The anti-inflammatory and soothing effects are achieved by reducing inflammatory factors; the inflammatory factors comprise IL-6.

The repairing of the damaged skin barriers is achieved by improving a cell migration rate and/or a relative cell proliferation rate.

The repairing of the damaged skin barriers includes repairing ultraviolet-induced skin barrier damage and/or trauma-induced skin barrier damage.

In an optional embodiment, the cells are human skin fibroblasts.

The present invention has the following beneficial effects:

In the present invention, PDRN is extracted from materials including *Lactobacillus sakei*, *Eubacterium rectale*, *Bacillus pumilus*, *Komagataella sp.*, *Anabaena variabilis* and guinea pigs for the first time, which addresses the drawbacks of salmon milt-derived PDRN such as unstable safety performance, poor eco-friendliness and insufficient sustainability, thereby expanding the scope of raw materials for PDRN preparation and providing multiple novel approaches for PDRN production.

The GC% of gDNA in PDRN prepared by the method described in the present invention has a similarity of over 99% with that of human gDNA, and is more approximate to human GC percentage than salmon-derived PDRN. Furthermore, PDRN with a GC content ranging from 40.5%-42% exhibits superior effects in promoting cell proliferation, boosting collagen synthesis, elevating cell migration rate, exerting anti-inflammatory and soothing effects, and/or repairing damaged skin barrier, compared with PDRN outside this GC range and salmon-derived PDRN. In addition, compared with the prior art extracting PDRN from salmon milt, the disclosed preparation method is simpler and more convenient to operate.

The present invention also provides a method for naturally extracting PDRN from raw materials including *Lactobacillus sakei*, *Eubacterium rectale*, *Bacillus pumilus*, *Komagataella sp.*, *Anabaena variabilis* and guinea pigs. The whole process of the method adopts closed-loop management with safe and controllable operation; meanwhile, no waste gas, waste water, waste residue or other pollutants are generated during production. Liquid residues such as fermentation broth and bacterial debris as well as solid residues can be processed into animal feeds. Therefore, the production process is eco-friendly and features excellent sustainability.

The yield of PDRN derived solely from salmon is prone to being limited by raw material supply, and the instability of the raw material further limits its scope of application. Salmon has a lengthy growth cycle of 3-7 years; in contrast, the multiple microbial strains in the present invention (*Lactobacillus sakei, Eubacterium rectale*, *Bacillus pumilus*, and *Komagataella sp.*) feature an extremely short fermentation cycle, while culture cycles for materials such as *Anabaena variabilis* and guinea pigs are also brief. This technology boasts numerous advantages including controllable growth conditions, pure target products, low production costs and outstanding sustainability. Furthermore, since *Lactobacillus sakei* is an edible strain, PDRN extracted therefrom can be applied in far broader scenarios in the future.

### BRIEF DESCRIPTION OF THE DRAWINGS

The above and/or additional aspects and advantages of the present invention will become apparent and readily understandable from the following description of the embodiments taken in conjunction with the accompanying drawings.
FIG. 1 is a test result graph of cytotoxicity;
FIG. 2 is a test result graph of UV repair efficacy;
FIG. 3 is a test result graph of trauma repair efficacy;
FIG. 4 is a test result graph of soothing efficacy;
FIG. 5 is a test result graph of collagen-promoting efficacy.

### Biological deposit information:

A strain of *Lactobacillus sake* LS-01 was deposited at the China Center for Type Culture Collection (CCTCC) on November 20, 2023, with the deposit number CCTCC NO: M 20232285. Deposit address: Wuhan University, Wuhan, China, Postal Code: 430072.

A strain of *Bacillus pumilus* Bp-1 was deposited at the China Center for Type Culture Collection (CCTCC) on February 12, 2025, with the deposit number CCTCC NO: M 2025198. Deposit address: Wuhan University, Wuhan, China, Postal Code: 430072.

A strain of *Bacillus pumilus* Bp-2 was deposited at the China Center for Type Culture Collection (CCTCC) on February 12, 2025, with the deposit number CCTCC NO: M 2025199. Deposit address: Wuhan University, Wuhan, China, Postal Code: 430072.

A strain of *Komagataella sp.* GS115 was deposited at the China Center for Type Culture Collection (CCTCC) on February 13, 2025, with the deposit number CCTCC NO: M 2025200. Deposit address: Wuhan University, Wuhan, China, Postal Code: 430072.

### DETAILED DESCRIPTION OF THE EMBODIMENTS

To more clearly illustrate the overall concept of the present invention, the present disclosure will be described in detail with reference to drawings by way of examples. In the description herein, a large number of specific details are given to provide a more thorough understanding of the present invention. However, it is obvious to those skilled in the art to implement the present invention without one or more details. In other examples, to avoid confusion with the present disclosure, certain technical features well known in the field are not described herein.

Where numerical ranges are provided in the embodiments, it shall be understood that both endpoints of each numerical range and any value falling between the two endpoints are optional unless otherwise specified in the present disclosure. Unless otherwise defined, all technical and scientific terms used in the present disclosure have the same meanings as commonly understood by those skilled in the art. In addition to the specific methods, apparatuses and materials employed in embodiments, any methods, apparatuses and materials of prior art similar or equivalent to those described in the embodiments of the present disclosure may be utilized to implement the present disclosure, based on the mastery of prior art by those skilled in the art and the recordation of the present disclosure.

As used in the present disclosure, the term polydeoxyribonucleotide (PDRN) refers to a high-molecular-weight double-stranded polymer or low-molecular-weight double-stranded polymer formed by repeating units composed of phosphoric acid, deoxyribose and four bases including adenine, guanine, thymine and cytosine.

Where specific conditions are not indicated in embodiments, operations are carried out under conventional conditions or conditions recommended by the manufacturer.

Unless otherwise specified, reagents or instruments used in the following embodiments without indicated manufacturers are conventional products commercially available.

Solid MRS medium (02-293) and liquid MRS medium (02-291) were purchased from Aoboxing Biotechnology (Beijing); DMEM medium (KGL1206-500) and RPMI-1640 medium (KGL1507-500) were purchased from KeyGen Biotech; *Lactobacillus rhamnosus* was purchased from the Institute of Microbiology, Chinese Academy of Sciences, with the deposit number CGMCC 1.8882; *Eubacterium rectale* ATCC 33656 was purchased from the American Type Culture Collection under the catalog number ATCC 33656; *Eubacterium rectale* BPB22 was purchased from the China Center for Type Culture Collection with the deposit number CCTCC NO: M 20232177; *Bacillus pumilus* Bp-1 was deposited at the China Center for Type Culture Collection under the deposit number CCTCC NO: M 2025198; *Bacillus pumilus* Bp-2 was deposited at the China Center for Type Culture Collection under the deposit number CCTCC NO: M 2025199; *Bacillus pumilus* ATCC 21143 was purchased from the American Type Culture Collection under the catalog number ATCC 21143; *Komagataella sp.* GS115 was deposited at the China Center for Type Culture Collection under the deposit number CCTCC NO: M 2025200; *Komagataella sp.* CICC 33158 was purchased from the China Center of Industrial Culture Collection with the catalog number CICC 33158; *Anabaena variabilis* was purchased from the American Type Culture Collection under the catalog number ATCC 29413; Guinea pigs (Cavia porcellus) of conventional grade were purchased from Liaoning Changsheng Biotechnology Co., Ltd. *Lactobacillus sakei* CICC 21858 with the deposit number CICC 21858 was purchased from the China Center of Industrial Culture Collection; Lysozyme (Cat. No. L1080) and snailase (Cat. No. S8280) were obtained from Beijing Solarbio Science & Technology Co., Ltd; the high-pressure homogenizer was manufactured by ATS with the model AH-BASIC 30; BCA protein quantification kit (Cat. No. E112-1) was purchased from Nanjing Vazyme Biotech Co., Ltd.

In addition, as described in the present disclosure, the term "water" encompasses any feasible water applicable in the cosmetic field, including deionized water, distilled water, ion-exchanged water, double-distilled water, high-purity water, purified water and the like.

In the following examples, unless otherwise specifically stated, "%" represents wt%, i.e., weight percentage.

### Example 1 Isolation and identification of Lactobacillus sakei

The pickle samples collected from Meishan, Sichuan were mashed under sterile conditions. After weighing, the samples were diluted with normal saline to prepare a 1% (w/v) solution. An appropriate volume of diluted solution was serially diluted 10-fold with normal saline. For each dilution gradient, 0.2 mL of diluted solution was aspirated and then coated onto a solid De Man, Rogosa and Sharpe Agar (MRS) agar plate, followed by inverted incubation at 37°C for 36-48 h. Milky white colonies with neat edges, smooth and glossy surfaces were selected and numbered 1-10 in descending order of colony size. Each of 10 single colonies described above was picked and inoculated into a liquid MRS medium, followed by static culture at 37°C for 48 h. Bacterial pellets were harvested separately, and a genomic DNA extraction kit was used to respectively perform genomic extraction.16S rDNA sequences were obtained via polymerase chain reaction (PCR) and Sanger sequencing by using genomic DNA as a template. Homology sequence alignment was performed through BLAST in the Nucleotide database of the National Center for Biotechnology Information (NCBI). The results indicated that strains No. 2, No. 7 and No. 9 were *Lactobacillus sakei*. Considering that the colony size reflects strain activity to a certain extent, *Lactobacillus sakei* strain No. 2 with the largest colony obtained in primary screening was selected as a strain for subsequent tests.

The 16S rDNA sequence of the *Lactobacillus sakei* strain No. 2 is shown in SEQ ID No. 1. The *Lactobacillus sakei* strain No. 2 was designated LS-01, which was deposited at the China Center for Type Culture Collection (CCTCC) on November 20, 2023, under the deposit number CCTCC NO: M 20232285. Deposit address: Wuhan University, Wuhan, Hubei, P.R. China, Postal Code: 430072.

### Example 2

### Test example 1

This example provides a preparation method of PDRN derived from *Lactobacillus sakei*, comprising the following steps:
Step 1, preparation of a fermentation broth via fermentation of *Lactobacillus sakei*:
The *Lactobacillus sake* LS-01 was cultured using the method described in Example 1. 1-2 loops of strains were picked from the solid MRS agar plate of the *Lactobacillus sake* LS-01 and inoculated into a 40 mL liquid MRS medium, followed by shake culture at 37°C and 160 rpm for 8 h to obtain a seed solution of *Lactobacillus sakei*. The seed solution of *Lactobacillus sakei* was inoculated into a 400 mL liquid MRS medium at an inoculation volume of 4%, followed by culturing at 37°C for 16 h to obtain a fermentation broth of *Lactobacillus sakei*.

Step 2, harvesting and resuspension of bacterial pellets and preparation of a bacterial lysate (300 mL of fermentation broth was taken as an example):
300 mL of bacterial suspension (fermentation broth of *Lactobacillus sakei*) was centrifuged at 9000 rpm for 5 min, and the supernatant was discarded. The bacterial pellets were rinsed once with 300 mL of TE buffer and centrifuged again at 9000 rpm for 5 min. 20 mL of TE buffer was added to resuspend the bacterial pellets evenly. Lysozyme was added to the TE-resuspended bacterial pellets to a final concentration of 0.2 mg/mL, followed by constant-temperature incubation at 37°C for 2 h. The mixture was subjected to high-pressure homogenization using a high-pressure homogenizer at a constant pressure of 1000 bar for 3 cycles to prepare the bacterial lysate.

Step 3, preparation of a salting-out supernatant via salting-out and centrifugation:
NaCl (with a final concentration of 2.5 M) and SDS (sodium dodecyl sulfate, with a final concentration of 1%) were added to the obtained bacterial lysate, followed by incubation at 60°C for 1.5 h. The supernatant was collected via centrifugation to prepare the salting-out supernatant.

Step 4, preparation of a crude product via ethanol precipitation:
Sodium acetate (with a final concentration of 0.3 M) was added to the salting-out supernatant obtained in step 3 and sufficiently stirred and evenly mixed; upon complete dissolution of sodium acetate, absolute ethanol with a volume 2.5 times that of the supernatant was slowly added to the supernatant for 30 min of precipitation. The mixture was centrifuged at 9000 rpm for 10 min, and the supernatant was discarded; the precipitate was rinsed once with 75% ethanol and then centrifuged at 9000 rpm for 10 min, and then the supernatant was discarded to obtain a wet product precipitate.

Step 5, obtaining of a product via dehydration and drying:
20 mL of absolute ethanol was added to the rinsed wet product precipitate for dehydration. After the precipitate was fully dispersed, the mixture was centrifuged at 9000 rpm for 10 min, and the supernatant was discarded (the operations were repeated 3 times). The resulting precipitate was transferred to a disposable culture dish and dried in vacuum at 40°C until the weight of the product no longer changed significantly (the weight variation of the product was less than 0.001 g). Upon completion of the drying, the PDRN product was obtained.

### Test example 2

Step 1, preparation of a fermentation broth via fermentation of *Lactobacillus sakei*:
The *Lactobacillus sake* LS-01 was cultured using the method described in Example 1. 1-2 loops of strains were picked from the solid MRS agar plate of the *Lactobacillus sake* LS-01 and inoculated into a 40 mL liquid MRS medium, followed by shake culture at 37°C and 160 rpm for 8 h to obtain a seed solution of *Lactobacillus sakei*. The seed solution of *Lactobacillus sakei* was inoculated into a 400 mL liquid MRS medium at an inoculation volume of 4%, followed by culturing at 37°C for 16 h to obtain a fermentation broth of *Lactobacillus sakei*.

Step 2, harvesting and resuspension of bacterial pellets and preparation of a bacterial lysate via high-pressure homogenization (300 mL of fermentation broth was taken as an example):
300 mL of bacterial suspension (fermentation broth of *Lactobacillus sakei*) was centrifuged at 9000 rpm for 5 min, and the supernatant was discarded. The bacterial pellets were rinsed once with 300 mL of TE buffer and centrifuged again at 9000 rpm for 5 min. 20 mL of TE buffer was added to resuspend the bacterial pellets evenly. Lysozyme was added to the TE-resuspended bacterial pellets to a final concentration of 0.2 mg/mL, followed by constant-temperature incubation at 37°C for 2 h. The mixture was subjected to high-pressure homogenization using a high-pressure homogenizer at a constant pressure of 1200 bar for 1 cycle to prepare the bacterial lysate.

Step 3, preparation of a salting-out supernatant via salting-out and centrifugation:
NaCl (with a final concentration of 1 M) and SDS (with a final concentration of 0.5%) were added to the obtained bacterial lysate, followed by incubation at 80°C for 1 h. The supernatant was collected via centrifugation to prepare the salting-out supernatant.

Step 4, preparation of a crude product via ethanol precipitation:
Sodium acetate (with a final concentration of 0.3 M) was added to the salting-out supernatant obtained in step 3 and sufficiently stirred and evenly mixed; upon complete dissolution of sodium acetate, absolute ethanol with a volume 2.5 times that of the supernatant was slowly added to the supernatant for 30 min of precipitation. The mixture was centrifuged at 9000 rpm for 10 min, and the supernatant was discarded; the precipitate was rinsed once with 75% ethanol and then centrifuged at 9000 rpm for 10 min, and then the supernatant was discarded to obtain a wet product precipitate.

Step 5, obtaining of a product via dehydration and drying:
20 mL of absolute ethanol was added to the rinsed wet product precipitate for dehydration. After the precipitate was fully dispersed, the mixture was centrifuged at 9000 rpm for 10 min, and the supernatant was discarded (the operations were repeated 3 times). The resulting precipitate was transferred to a disposable culture dish and dried in vacuum at 40°C until the weight of the product no longer changed significantly (the weight variation of the product was less than 0.001 g). Upon completion of the drying, the PDRN product was obtained.

### Test example 3

Step 1, preparation of a fermentation broth via fermentation of *Lactobacillus sakei*:
The *Lactobacillus sake LS-01* was cultured using the method described in Example 1. 1-2 loops of strains were picked from the solid MRS agar plate of the *Lactobacillus sake LS-01* and inoculated into a 40 mL liquid MRS medium, followed by shake culture at 37°C and 160 rpm for 8 h to obtain a seed solution of *Lactobacillus sakei*. The seed solution of *Lactobacillus sakei* was inoculated into a 400 mL liquid MRS medium at an inoculation volume of 4%, followed by culturing at 37°C for 16 h to obtain a fermentation broth of *Lactobacillus sakei*.

Step 2, harvesting and resuspension of bacterial pellets and preparation of a bacterial lysate via high-pressure homogenization (300 mL of fermentation broth was taken as an example):
300 mL of bacterial suspension (fermentation broth of *Lactobacillus sakei*) was centrifuged at 9000 rpm for 5 min, and the supernatant was discarded. The bacterial pellets were rinsed once with 300 mL of TE buffer and centrifuged again at 9000 rpm for 5 min. 20 mL of TE buffer was added to resuspend the bacterial pellets evenly. Lysozyme was added to the TE-resuspended bacterial pellets to a final concentration of 0.2 mg/mL, followed by constant-temperature incubation at 37°C for 2 h. The mixture was subjected to high-pressure homogenization using a high-pressure homogenizer at a constant pressure of 900 bar for 4 cycles to prepare the bacterial lysate.

Step 3, preparation of a salting-out supernatant via salting-out and centrifugation:
NaCl (with a final concentration of 4 M) and SDS (with a final concentration of 1.5%) were added to the obtained bacterial lysate, followed by incubation at 50°C for 2.5 h. The supernatant was collected via centrifugation to prepare the salting-out supernatant.

Step 4, preparation of a crude product via ethanol precipitation:
Sodium acetate (with a final concentration of 0.3 M) was added to the salting-out supernatant obtained in step 3 and sufficiently stirred and evenly mixed; upon complete dissolution of sodium acetate, absolute ethanol with a volume 2.5 times that of the supernatant was slowly added to the supernatant for 30 min of precipitation. The mixture was centrifuged at 9000 rpm for 10 min, and the supernatant was discarded; the precipitate was rinsed once with 75% ethanol and then centrifuged at 9000 rpm for 10 min, and then the supernatant was discarded to obtain a wet product precipitate.

Step 5, obtaining of a product via dehydration and drying:
20 mL of absolute ethanol was added to the rinsed wet product precipitate for dehydration. After the precipitate was fully dispersed, the mixture was centrifuged at 9000 rpm for 10 min, and the supernatant was discarded (the operations were repeated 3 times). The resulting precipitate was transferred to a disposable culture dish and dried in vacuum at 40°C until the weight of the product no longer changed significantly (the weight variation of the product was less than 0.001 g). Upon completion of the drying, the PDRN product was obtained.

### Test example 4

Step 1, preparation of a fermentation broth via fermentation of Lactobacillus sakei:
The Lactobacillus sake LS-01 was cultured using the method described in Example 1. 1-2 loops of strains were picked from the solid MRS agar plate of the *Lactobacillus sake* LS-01 and inoculated into a 40 mL liquid MRS medium, followed by shake culture at 37°C and 160 rpm for 8 h to obtain a seed solution of *Lactobacillus sakei*. The seed solution of *Lactobacillus sakei* was inoculated into a 400 mL liquid MRS medium at an inoculation volume of 4%, followed by culturing at 37°C for 16 h to obtain a fermentation broth of *Lactobacillus sakei*.

Step 2, harvesting and resuspension of bacterial pellets and preparation of a bacterial lysate via high-pressure homogenization (300 mL of fermentation broth was taken as an example):
300 mL of bacterial suspension (fermentation broth of *Lactobacillus sakei*) was centrifuged at 9000 rpm for 5 min, and the supernatant was discarded. The bacterial pellets were rinsed once with 300 mL of TE buffer and centrifuged again at 9000 rpm for 5 min. 20 mL of TE buffer was added to resuspend the bacterial pellets evenly. Lysozyme was added to the TE-resuspended bacterial pellets to a final concentration of 0.4 mg/mL, followed by constant-temperature incubation at 37°C for 2 h. The mixture was subjected to high-pressure homogenization using a high-pressure homogenizer at a constant pressure of 800 bar for 2 cycles to prepare the bacterial lysate.

Step 3, preparation of a salting-out supernatant via salting-out and centrifugation:
NaCl (with a final concentration of 2.5 M) and SDS (with a final concentration of 1%) were added to the obtained bacterial lysate, followed by incubation at 60°C for 1.5 h. The supernatant was collected via centrifugation to prepare the salting-out supernatant.

Step 4, preparation of a crude product via ethanol precipitation:
Sodium acetate (with a final concentration of 0.3 M) was added to the salting-out supernatant obtained in step 3 and sufficiently stirred and evenly mixed; upon complete dissolution of sodium acetate, absolute ethanol with a volume 2.5 times that of the supernatant was slowly added to the supernatant for 30 min of precipitation. The mixture was centrifuged at 9000 rpm for 10 min, and the supernatant was discarded; the precipitate was rinsed once with 75% ethanol and then centrifuged at 9000 rpm for 10 min, and then the supernatant was discarded to obtain a wet product precipitate.

Step 5, obtaining of a product via dehydration and drying:
20 mL of absolute ethanol was added to the rinsed wet product precipitate for dehydration. After the precipitate was fully dispersed, the mixture was centrifuged at 9000 rpm for 10 min, and the supernatant was discarded (the operations were repeated 3 times). The resulting precipitate was transferred to a disposable culture dish and dried in vacuum at 40°C until the weight of the product no longer changed significantly (the weight variation of the product was less than 0.001 g). Upon completion of the drying, the PDRN product was obtained.

### Test example 5

Step 1, preparation of a fermentation broth via fermentation of *Lactobacillus sakei*:
The *Lactobacillus sake* LS-01 was cultured using the method described in Example 1. 1-2 loops of strains were picked from the solid MRS agar plate of the *Lactobacillus sake* LS-01 and inoculated into a 40 mL liquid MRS medium, followed by shake culture at 37°C and 160 rpm for 8 h to obtain a seed solution of *Lactobacillus sakei*. The seed solution of *Lactobacillus sakei* was inoculated into a 400 mL liquid MRS medium at an inoculation volume of 4%, followed by culturing at 37°C for 16 h to obtain a fermentation broth of *Lactobacillus sakei*.

Step 2, harvesting and resuspension of bacterial pellets and preparation of a bacterial lysate via high-pressure homogenization (300 mL of fermentation broth was taken as an example):
300 mL of bacterial suspension (fermentation broth of *Lactobacillus sakei*) was centrifuged at 9000 rpm for 5 min, and the supernatant was discarded. The bacterial pellets were rinsed once with 300 mL of TE buffer and centrifuged again at 9000 rpm for 5 min. 20 mL of TE buffer was added to resuspend the bacterial pellets evenly. Lysozyme was added to the TE-resuspended bacterial pellets to a final concentration of 0.4 mg/mL, followed by constant-temperature incubation at 37°C for 4 h. The mixture was subjected to high-pressure homogenization using a high-pressure homogenizer at a constant pressure of 800 bar for 1 cycle to prepare the bacterial lysate.

Step 3, preparation of a salting-out supernatant via salting-out and centrifugation:
NaCl (with a final concentration of 2.5 M) and SDS (with a final concentration of 1%) were added to the obtained bacterial lysate, followed by incubation at 60°C for 1.5 h. The supernatant was collected via centrifugation to prepare the salting-out supernatant.

Step 4, preparation of a crude product via ethanol precipitation:
Sodium acetate (with a final concentration of 0.3 M) was added to the salting-out supernatant obtained in step 3 and sufficiently stirred and evenly mixed; upon complete dissolution of sodium acetate, absolute ethanol with a volume 2.5 times that of the supernatant was slowly added to the supernatant for 30 min of precipitation. The mixture was centrifuged at 9000 rpm for 10 min, and the supernatant was discarded; the precipitate was rinsed once with 75% ethanol and then centrifuged at 9000 rpm for 10 min, and then the supernatant was discarded to obtain a wet product precipitate.

Step 5, obtaining of a product via dehydration and drying:
20 mL of absolute ethanol was added to the rinsed wet product precipitate for dehydration. After the precipitate was fully dispersed, the mixture was centrifuged at 9000 rpm for 10 min, and the supernatant was discarded (the operations were repeated 3 times). The resulting precipitate was transferred to a disposable culture dish and dried in vacuum at 40°C until the weight of the product no longer changed significantly (the weight variation of the product was less than 0.001 g). Upon completion of the drying, the PDRN product was obtained.

### Test example 6

This test example is different from Test example 1 only in that the fermentation strain used is Lactobacillus *sakei* CICC21858 with the deposit number CICC21858, which was purchased from the China Center of Industrial Culture Collection.

Step 1, preparation of a fermentation broth via fermentation of *Lactobacillus sakei*:
The *Lactobacillus sakei* CICC21858 was cultured according to the method in Example 1, 1-2 loops of strains were picked from the solid MRS agar plate of the *Lactobacillus sakei* CICC21858 and inoculated into a 40 mL liquid MRS medium, followed by shake culture at 37°C and 160 rpm for 8 h to obtain a seed solution of *Lactobacillus sakei*. The seed solution of *Lactobacillus sakei* was inoculated into a 400 mL liquid MRS medium at an inoculation volume of 4%, followed by culturing at 37°C for 16 h to obtain a fermentation broth of *Lactobacillus sakei*.

Step 2, harvesting and resuspension of bacterial pellets and preparation of a bacterial lysate (300 mL of fermentation broth was taken as an example):
300 mL of bacterial suspension (fermentation broth of *Lactobacillus sakei*) was centrifuged at 9000 rpm for 5 min, and the supernatant was discarded. The bacterial pellets were rinsed once with 300 mL of TE buffer and centrifuged again at 9000 rpm for 5 min. 20 mL of TE buffer was added to resuspend the bacterial pellets evenly. Lysozyme was added to the TE-resuspended bacterial pellets to a final concentration of 0.2 mg/mL, followed by constant-temperature incubation at 37°C for 2 h. The mixture was subjected to high-pressure homogenization using a high-pressure homogenizer at a constant pressure of 1000 bar for 3 cycles to prepare the bacterial lysate.

Step 3, preparation of a salting-out supernatant via salting-out and centrifugation:
NaCl (with a final concentration of 2.5 M) and SDS (sodium dodecyl sulfate, with a final concentration of 1%) were added to the obtained bacterial lysate, followed by incubation at 60°C for 1.5 h. The supernatant was collected via centrifugation to prepare the salting-out supernatant.

Step 4, preparation of a crude product via ethanol precipitation:
Sodium acetate (with a final concentration of 0.3 M) was added to the salting-out supernatant obtained in step 3 and sufficiently stirred and evenly mixed; upon complete dissolution of sodium acetate, absolute ethanol with a volume 2.5 times that of the supernatant was slowly added to the supernatant for 30 min of precipitation. The mixture was centrifuged at 9000 rpm for 10 min, and the supernatant was discarded; the precipitate was rinsed once with 75% ethanol and then centrifuged at 9000 rpm for 10 min, and then the supernatant was discarded to obtain a wet product precipitate.

Step 5, obtaining of a product via dehydration and drying:
20 mL of absolute ethanol was added to the rinsed wet product precipitate for dehydration. After the precipitate was fully dispersed, the mixture was centrifuged at 9000 rpm for 10 min, and the supernatant was discarded (the operations were repeated 3 times). The resulting precipitate was transferred to a disposable culture dish and dried in vacuum at 40°C until the weight of the product no longer changed significantly (the weight variation of the product was less than 0.001 g). Upon completion of the drying, the PDRN product was obtained.

### Test example 7

This test example provides a preparation method of PDRN derived from *Lactobacillus rhamnosus* (with the deposit number CGMCC 1.8882), specifically comprising the following steps:
Step 1, preparation of a fermentation broth via fermentation of *Lactobacillus rhamnosus*:
   1-2 loops of strains were picked from a solid MRS agar plate for culturing *Lactobacillus* rhamnosus and inoculated into a 40 mL liquid MRS medium, followed by anaerobic culture at 37°C for 8 h to obtain a seed solution of *Lactobacillus rhamnosus*. The seed solution of *Lactobacillus rhamnosus* was inoculated into a 400 mL liquid MRS medium at an inoculation volume of 4%, followed by culturing at 37°C for 16 h to obtain a fermentation broth of *Lactobacillus rhamnosus*.
Step 2, harvesting and resuspension of bacterial pellets and preparation of a bacterial lysate via high-pressure homogenization (300 mL of fermentation broth was taken as an example):
   300 mL of bacterial suspension (fermentation broth of *Lactobacillus rhamnosus*) was centrifuged at 9000 rpm for 5 min, and the supernatant was discarded. The bacterial pellets were rinsed once with 300 mL of TE buffer and centrifuged again at 9000 rpm for 5 min. 20 mL of TE buffer was added to resuspend the bacterial pellets evenly. Lysozyme was added to the TE-resuspended bacterial pellets to a final concentration of 0.2 mg/mL, followed by constant-temperature incubation at 37°C for 2 h. The mixture was subjected to high-pressure homogenization using a high-pressure homogenizer at a constant pressure of 1000 bar for 3 cycles to prepare the bacterial lysate.
Step 3, preparation of a salting-out supernatant via salting-out and centrifugation:
   NaCl (with a final concentration of 2.5 M) and SDS (with a final concentration of 1%) were added to the obtained bacterial lysate, followed by incubation at 60°C for 1.5 h. The supernatant was collected via centrifugation to prepare the salting-out supernatant.
Step 4, preparation of a crude product via ethanol precipitation:
   Sodium acetate (with a final concentration of 0.3 M) was added to the salting-out supernatant obtained in step 3 and sufficiently stirred and evenly mixed; upon complete dissolution of sodium acetate, absolute ethanol with a volume 2.5 times that of the supernatant was slowly added to the supernatant for 30 min of precipitation. The mixture was centrifuged at 9000 rpm for 10 min, and the supernatant was discarded; the precipitate was rinsed once with 75% ethanol and then centrifuged at 9000 rpm for 10 min, and then the supernatant was discarded to obtain a wet product precipitate.
Step 5, obtaining of a product via dehydration and drying:
   20 mL of absolute ethanol was added to the rinsed wet product precipitate for dehydration. After the precipitate was fully dispersed, the mixture was centrifuged at 9000 rpm for 10 min, and the supernatant was discarded (the operations were repeated 3 times). The resulting precipitate was transferred to a disposable culture dish and dried in vacuum at 40°C until the weight of the product no longer changed significantly (the weight variation of the product was less than 0.001 g). Upon completion of the drying, the PDRN product was obtained.

### Test example 8

This test example provides a preparation method of PDRN derived from *Eubacterium rectale* ATCC 33656 (purchased from American Type Culture Collection under the deposit number ATCC 33656), specifically comprising the following steps:
Step 1, preparation of a fermentation broth via fermentation of *Eubacterium rectale*:
   1-2 loops of strains were picked from a solid PYG agar plate (purchased from Beijing Solarbio Science & Technology Co., Ltd., Cat. No. LA5570) for culturing *Eubacterium rectale* ATCC 33656 and inoculated into a 40 mL liquid PYG medium, followed by anaerobic culture at 37°C for 16 h to obtain a seed solution of *Eubacterium rectale*. The seed solution of *Eubacterium rectale* was inoculated into a 400 mL liquid PYG medium at an inoculation volume of 4%, followed by anaerobic culture at 37°C for 48 h to obtain a fermentation broth of *Eubacterium rectale*.
Step 2, harvesting and resuspension of bacterial pellets and preparation of a bacterial lysate (300 mL of fermentation broth was taken as an example):
   300 mL of bacterial suspension (fermentation broth of *Eubacterium rectale*) was centrifuged at 9000 rpm for 5 min, and the supernatant was discarded. The bacterial pellets were rinsed once with 300 mL of TE buffer and centrifuged again at 9000 rpm for 5 min. 20 mL of TE buffer was added to resuspend the bacterial pellets evenly. Lysozyme was added to the TE-resuspended bacterial pellets to a final concentration of 0.2 mg/mL, followed by constant-temperature incubation at 37°C for 2 h. The mixture was subjected to high-pressure homogenization using a high-pressure homogenizer at a constant pressure of 1000 bar for 3 cycles to prepare the bacterial lysate.
Step 3, preparation of a salting-out supernatant via salting-out and centrifugation:
   NaCl (with a final concentration of 2.5 M) and SDS (sodium dodecyl sulfate, with a final concentration of 1%) were added to the obtained bacterial lysate, followed by incubation at 60°C for 1.5 h. The supernatant was collected via centrifugation to prepare the salting-out supernatant.
Step 4, preparation of a crude product via ethanol precipitation:
   Sodium acetate (with a final concentration of 0.3 M) was added to the salting-out supernatant obtained in step 3 and sufficiently stirred and evenly mixed; upon complete dissolution of sodium acetate, absolute ethanol with a volume 2.5 times that of the supernatant was slowly added to the supernatant for 30 min of precipitation. The mixture was centrifuged at 9000 rpm for 10 min, and the supernatant was discarded; the precipitate was rinsed once with 75% ethanol and then centrifuged at 9000 rpm for 10 min, and then the supernatant was discarded to obtain a wet product precipitate.
Step 5, obtaining of a product via dehydration and drying:
   20 mL of absolute ethanol was added to the rinsed precipitate for dehydration. After the precipitate was fully dispersed, the mixture was centrifuged at 9000 rpm for 10 min, and the supernatant was discarded (the operations were repeated 3 times). The resulting precipitate was transferred to a disposable culture dish and dried in vacuum at 40°C until the weight of the product no longer changed significantly (the weight variation of the product was less than 0.001 g). Upon completion of the drying, the PDRN product was obtained.

### Test example 9

This test example provides a preparation method of PDRN derived from *Eubacterium rectale* BPB22 (purchased from the China Center for Type Culture Collection under the deposit number CCTCC NO: M 20232177), specifically comprising the following steps:
Step 1, preparation of a fermentation broth via fermentation of *Eubacterium rectale*:
   1-2 loops of strains were picked from a solid PYG agar plate (purchased from Beijing Solarbio Science & Technology Co., Ltd., Cat. No. LA5570) for culturing *Eubacterium rectale* BPB22 and inoculated into a 40 mL liquid PYG medium, followed by anaerobic culture at 37°C for 16 h to obtain a seed solution of *Eubacterium rectale*. The seed solution of *Eubacterium rectale* was inoculated into a 400 mL liquid PYG medium at an inoculation volume of 4%, followed by anaerobic culture at 37°C for 48 h to obtain a fermentation broth of *Eubacterium rectale*.
Step 2, harvesting and resuspension of bacterial pellets and preparation of a bacterial lysate (300 mL of fermentation broth was taken as an example):
   300 mL of bacterial suspension (fermentation broth of *Eubacterium rectale*) was centrifuged at 9000 rpm for 5 min, and the supernatant was discarded. The bacterial pellets were rinsed once with 300 mL of TE buffer and centrifuged again at 9000 rpm for 5 min. 20 mL of TE buffer was added to resuspend the bacterial pellets evenly. Lysozyme was added to the TE-resuspended bacterial pellets to a final concentration of 0.2 mg/mL, followed by constant-temperature incubation at 37°C for 2 h. The mixture was subjected to high-pressure homogenization using a high-pressure homogenizer at a constant pressure of 1000 bar for 3 cycles to prepare the bacterial lysate.
Step 3, preparation of a salting-out supernatant via salting-out and centrifugation:
   NaCl (with a final concentration of 2.5 M) and SDS (sodium dodecyl sulfate, with a final concentration of 1%) were added to the obtained bacterial lysate, followed by incubation at 60°C for 1.5 h. The supernatant was collected via centrifugation to prepare the salting-out supernatant.
Step 4, preparation of a crude product via ethanol precipitation:
   Sodium acetate (with a final concentration of 0.3 M) was added to the salting-out supernatant obtained in step 3 and sufficiently stirred and evenly mixed; upon complete dissolution of sodium acetate, absolute ethanol with a volume 2.5 times that of the supernatant was slowly added to the supernatant for 30 min of precipitation. The mixture was centrifuged at 9000 rpm for 10 min, and the supernatant was discarded; the precipitate was rinsed once with 75% ethanol and then centrifuged at 9000 rpm for 10 min, and then the supernatant was discarded to obtain a wet product precipitate.
Step 5, obtaining of a product via dehydration and drying:
   20 mL of absolute ethanol was added to the rinsed precipitate for dehydration. After the precipitate was fully dispersed, the mixture was centrifuged at 9000 rpm for 10 min, and the supernatant was discarded (the operations were repeated 3 times). The resulting precipitate was transferred to a disposable culture dish and dried in vacuum at 40°C until the weight of the product no longer changed significantly (the weight variation of the product was less than 0.001 g). Upon completion of the drying, the PDRN product was obtained.

### Test example 10

This test example provides a preparation method of PDRN derived from *Bacillus pumilus* Bp-1 (deposited at the China Center for Type Culture Collection under the deposit number CCTCC NO: M 2025198), specifically comprising the following steps:
Step 1, preparation of a fermentation broth via fermentation of *Bacillus pumilus*:
   1-2 loops of strains were picked from a solid LB agar plate (purchased from Beijing Solarbio Science & Technology Co., Ltd., Cat. No. L1010) for culturing *Bacillus pumilus* Bp-1 and inoculated into a 40 mL liquid LB medium, followed by culturing at 37°C and 150 rpm for 12 h to obtain a seed solution of *Bacillus pumilus*. The seed solution of *Bacillus pumilus* was inoculated into a 400 mL liquid LB medium at an inoculation volume of 4%, followed by culturing at 37°C and 150 rpm for 24 h to obtain a fermentation broth of *Bacillus pumilus*.
Step 2, harvesting and resuspension of bacterial pellets and preparation of a bacterial lysate (300 mL of fermentation broth was taken as an example):
   300 mL of bacterial suspension (fermentation broth of *Bacillus pumilus*) was centrifuged at 9000 rpm for 5 min, and the supernatant was discarded. The bacterial pellets were rinsed once with 300 mL of TE buffer and centrifuged again at 9000 rpm for 5 min. 20 mL of TE buffer was added to resuspend the bacterial pellets evenly. Lysozyme was added to the TE-resuspended bacterial pellets to a final concentration of 0.2 mg/mL, followed by constant-temperature incubation at 37°C for 2 h. The mixture was subjected to high-pressure homogenization using a high-pressure homogenizer at a constant pressure of 1000 bar for 3 cycles to prepare the bacterial lysate.
Step 3, preparation of a salting-out supernatant via salting-out and centrifugation:
   NaCl (with a final concentration of 2.5 M) and SDS (sodium dodecyl sulfate, with a final concentration of 1%) were added to the obtained bacterial lysate, followed by incubation at 60°C for 1.5 h. The supernatant was collected via centrifugation to prepare the salting-out supernatant.
Step 4, preparation of a crude product via ethanol precipitation:
   Sodium acetate (with a final concentration of 0.3 M) was added to the salting-out supernatant obtained in step 3 and sufficiently stirred and evenly mixed; upon complete dissolution of sodium acetate, absolute ethanol with a volume 2.5 times that of the supernatant was slowly added to the supernatant for 30 min of precipitation. The mixture was centrifuged at 9000 rpm for 10 min, and the supernatant was discarded; the precipitate was rinsed once with 75% ethanol and then centrifuged at 9000 rpm for 10 min, and then the supernatant was discarded to obtain a wet product precipitate.
Step 5, obtaining of a product via dehydration and drying:
   20 mL of absolute ethanol was added to the rinsed precipitate for dehydration. After the precipitate was fully dispersed, the mixture was centrifuged at 9000 rpm for 10 min, and the supernatant was discarded (the operations were repeated 3 times). The resulting precipitate was transferred to a disposable culture dish and dried in vacuum at 40°C until the weight of the product no longer changed significantly (the weight variation of the product was less than 0.001 g). Upon completion of the drying, the PDRN product was obtained.

### Test example 11

This test example provides a preparation method of PDRN derived from *Bacillus pumilus* Bp-2 (deposited at the China Center for Type Culture Collection under the deposit number CCTCC NO: M 2025199), specifically comprising the following steps:
Step 1, preparation of a fermentation broth via fermentation of Bacillus pumilus:
   1-2 loops of strains were picked from a solid LB agar plate (purchased from Beijing Solarbio Science & Technology Co., Ltd., Cat. No. L1010) for culturing Bacillus pumilus Bp-2 and inoculated into a 40 mL liquid LB medium, followed by culturing at 37°C and 150 rpm for 12 h to obtain a seed solution of Bacillus pumilus. The seed solution of *Bacillus pumilus* was inoculated into a 400 mL liquid LB medium at an inoculation volume of 4%, followed by culturing at 37°C and 150 rpm for 24 h to obtain a fermentation broth of *Bacillus pumilus*.
Step 2, harvesting and resuspension of bacterial pellets and preparation of a bacterial lysate (300 mL of fermentation broth was taken as an example):
   300 mL of bacterial suspension (fermentation broth of *Bacillus pumilus*) was centrifuged at 9000 rpm for 5 min, and the supernatant was discarded. The bacterial pellets were rinsed once with 300 mL of TE buffer and centrifuged again at 9000 rpm for 5 min. 20 mL of TE buffer was added to resuspend the bacterial pellets evenly. Lysozyme was added to the TE-resuspended bacterial pellets to a final concentration of 0.2 mg/mL, followed by constant-temperature incubation at 37°C for 2 h. The mixture was subjected to high-pressure homogenization using a high-pressure homogenizer at a constant pressure of 1000 bar for 3 cycles to prepare the bacterial lysate.
Step 3, preparation of a salting-out supernatant via salting-out and centrifugation:
   NaCl (with a final concentration of 2.5 M) and SDS (sodium dodecyl sulfate, with a final concentration of 1%) were added to the obtained bacterial lysate, followed by incubation at 60°C for 1.5 h. The supernatant was collected via centrifugation to prepare the salting-out supernatant.
Step 4, preparation of a crude product via ethanol precipitation:
   Sodium acetate (with a final concentration of 0.3 M) was added to the salting-out supernatant obtained in step 3 and sufficiently stirred and evenly mixed; upon complete dissolution of sodium acetate, absolute ethanol with a volume 2.5 times that of the supernatant was slowly added to the supernatant for 30 min of precipitation. The mixture was centrifuged at 9000 rpm for 10 min, and the supernatant was discarded; the precipitate was rinsed once with 75% ethanol and then centrifuged at 9000 rpm for 10 min, and then the supernatant was discarded to obtain a wet product precipitate.
Step 5, obtaining of a product via dehydration and drying:
   20 mL of absolute ethanol was added to the rinsed precipitate for dehydration. After the precipitate was fully dispersed, the mixture was centrifuged at 9000 rpm for 10 min, and the supernatant was discarded (the operations were repeated 3 times). The resulting precipitate was transferred to a disposable culture dish and dried in vacuum at 40°C until the weight of the product no longer changed significantly (the weight variation of the product was less than 0.001 g). Upon completion of the drying, the PDRN product was obtained.

### Test example 12

This test example provides a preparation method of PDRN derived from *Bacillus pumilus* ATCC 21143 (purchased from the American Type Culture Collection under the deposit number ATCC 21143), specifically comprising the following steps:
Step 1, preparation of a fermentation broth via fermentation of *Bacillus pumilus*:
   1-2 loops of strains were picked from a solid LB agar plate (purchased from Beijing Solarbio Science & Technology Co., Ltd., Cat. No. L1010) for culturing *Bacillus pumilus* ATCC 21143 and inoculated into a 40 mL liquid LB medium, followed by culturing at 37°C and 150 rpm for 12 h to obtain a seed solution of *Bacillus pumilus*. The seed solution of *Bacillus pumilus* was inoculated into a 400 mL liquid LB medium at an inoculation volume of 4%, followed by culturing at 37°C and 150 rpm for 24 h to obtain a fermentation broth of *Bacillus pumilus*.
Step 2, harvesting and resuspension of bacterial pellets and preparation of a bacterial lysate (300 mL of fermentation broth was taken as an example):
   300 mL of bacterial suspension (fermentation broth of *Bacillus pumilus*) was centrifuged at 9000 rpm for 5 min, and the supernatant was discarded. The bacterial pellets were rinsed once with 300 mL of TE buffer and centrifuged again at 9000 rpm for 5 min. 20 mL of TE buffer was added to resuspend the bacterial pellets evenly. Lysozyme was added to the TE-resuspended bacterial pellets to a final concentration of 0.2 mg/mL, followed by constant-temperature incubation at 37°C for 2 h. The mixture was subjected to high-pressure homogenization using a high-pressure homogenizer at a constant pressure of 1000 bar for 3 cycles to prepare the bacterial lysate.
Step 3, preparation of a salting-out supernatant via salting-out and centrifugation:
   NaCl (with a final concentration of 2.5 M) and SDS (sodium dodecyl sulfate, with a final concentration of 1%) were added to the obtained bacterial lysate, followed by incubation at 60°C for 1.5 h. The supernatant was collected via centrifugation to prepare the salting-out supernatant.
Step 4, preparation of a crude product via ethanol precipitation:
   Sodium acetate (with a final concentration of 0.3 M) was added to the salting-out supernatant obtained in step 3 and sufficiently stirred and evenly mixed; upon complete dissolution of sodium acetate, absolute ethanol with a volume 2.5 times that of the supernatant was slowly added to the supernatant for 30 min of precipitation. The mixture was centrifuged at 9000 rpm for 10 min, and the supernatant was discarded; the precipitate was rinsed once with 75% ethanol and then centrifuged at 9000 rpm for 10 min, and then the supernatant was discarded to obtain a wet product precipitate.
Step 5, obtaining of a product via dehydration and drying:
   20 mL of absolute ethanol was added to the rinsed precipitate for dehydration. After the precipitate was fully dispersed, the mixture was centrifuged at 9000 rpm for 10 min, and the supernatant was discarded (the operations were repeated 3 times). The resulting precipitate was transferred to a disposable culture dish and dried in vacuum at 40°C until the weight of the product no longer changed significantly (the weight variation of the product was less than 0.001 g). Upon completion of the drying, the PDRN product was obtained.

### Test example 13

This test example provides a preparation method of PDRN derived from *Komagataella sp.* GS115 (deposited at the China Center for Type Culture Collection under the deposit number CCTCC NO: M 2025200), specifically comprising the following steps:
Step 1, preparation of a fermentation broth via fermentation of *Komagataella sp.*:
   1-2 loops of strains were picked from a solid YPD agar plate (purchased from Beijing Solarbio Science & Technology Co., Ltd., Cat. No. LA0220) for culturing *Komagataella sp.* GS115 and inoculated into a 40 mL liquid YPD medium, followed by shake culture at 30°C and 220 rpm for 16 h to obtain a seed solution of *Komagataella sp.* The seed solution of *Komagataella sp.* was inoculated into a 400 mL liquid YPD medium at an inoculation volume of 4%, followed by culturing at 30°C and 220 rpm for 30 h to obtain a fermentation broth of *Komagataella sp.*
Step 2, harvesting and resuspension of bacterial pellets and preparation of a bacterial lysate (300 mL of fermentation broth was taken as an example):
   300 mL of bacterial suspension (fermentation broth of *Komagataella sp.*) was centrifuged at 9000 rpm for 5 min, and the supernatant was discarded. The bacterial pellets were rinsed once with 300 mL of TE buffer and centrifuged again at 9000 rpm for 5 min. 20 mL of TE buffer was added to resuspend the bacterial pellets evenly. Snailase (purchased from Beijing Solarbio Science & Technology Co., Ltd., Cat. No. S8280) was added to the TE-resuspended bacterial pellets to a final concentration of 0.2 mg/mL, followed by constant-temperature incubation at 45°C for 2 h. The mixture was subjected to high-pressure homogenization using a high-pressure homogenizer at a constant pressure of 1000 bar for 3 cycles to prepare the bacterial lysate.
Step 3, preparation of a salting-out supernatant via salting-out and centrifugation:
   NaCl (with a final concentration of 2.5 M) and SDS (sodium dodecyl sulfate, with a final concentration of 1%) were added to the obtained bacterial lysate, followed by incubation at 60°C for 1.5 h. The supernatant was collected via centrifugation to prepare the salting-out supernatant.
Step 4, preparation of a crude product via ethanol precipitation:
   Sodium acetate (with a final concentration of 0.3 M) was added to the salting-out supernatant obtained in step 3 and sufficiently stirred and evenly mixed; upon complete dissolution of sodium acetate, absolute ethanol with a volume 2.5 times that of the supernatant was slowly added to the supernatant for 30 min of precipitation. The mixture was centrifuged at 9000 rpm for 10 min, and the supernatant was discarded; the precipitate was rinsed once with 75% ethanol and then centrifuged at 9000 rpm for 10 min, and then the supernatant was discarded to obtain a wet product precipitate.
Step 5, obtaining of a product via dehydration and drying:
   20 mL of absolute ethanol was added to the rinsed precipitate for dehydration. After the precipitate was fully dispersed, the mixture was centrifuged at 9000 rpm for 10 min, and the supernatant was discarded (the operations were repeated 3 times). The resulting precipitate was transferred to a disposable culture dish and dried in vacuum at 40°C until the weight of the product no longer changed significantly (the weight variation of the product was less than 0.001 g). Upon completion of the drying, the PDRN product was obtained.

### Test example 14

This test example provides a preparation method of PDRN derived from *Komagataella sp.* CICC 33158 (purchased from the China Center of Industrial Culture Collection under the deposit number CICC 33158), specifically comprising the following steps:
Step 1, preparation of a fermentation broth via fermentation of *Komagataella sp.*:
   1-2 loops of strains were picked from a solid YPD agar plate (purchased from Beijing Solarbio Science & Technology Co., Ltd., Cat. No. LA0220) for culturing *Komagataella sp.* and inoculated into a 40 mL liquid YPD medium, followed by shake culture at 30°C and 220 rpm for 16 h to obtain a seed solution of *Komagataella sp.* The seed solution of *Komagataella sp.* was inoculated into a 400 mL liquid YPD medium at an inoculation volume of 4%, followed by culturing at 30°C and 220 rpm for 30 h to obtain a fermentation broth of *Komagataella sp.*
Step 2, harvesting and resuspension of bacterial pellets and preparation of a bacterial lysate (300 mL of fermentation broth was taken as an example):
   300 mL of bacterial suspension (fermentation broth of *Komagataella sp.*) was centrifuged at 9000 rpm for 5 min, and the supernatant was discarded. The bacterial pellets were rinsed once with 300 mL of TE buffer and centrifuged again at 9000 rpm for 5 min. 20 mL of TE buffer was added to resuspend the bacterial pellets evenly. Snailase (purchased from Beijing Solarbio Science & Technology Co., Ltd., Cat. No. S8280) was added to the TE-resuspended bacterial pellets to a final concentration of 0.2 mg/mL, followed by constant-temperature incubation at 45°C for 2 h. The mixture was subjected to high-pressure homogenization using a high-pressure homogenizer at a constant pressure of 1000 bar for 3 cycles to prepare the bacterial lysate.
Step 3, preparation of a salting-out supernatant via salting-out and centrifugation:
   NaCl (with a final concentration of 2.5 M) and SDS (sodium dodecyl sulfate, with a final concentration of 1%) were added to the obtained bacterial lysate, followed by incubation at 60°C for 1.5 h. The supernatant was collected via centrifugation to prepare the salting-out supernatant.
Step 4, preparation of a crude product via ethanol precipitation:
   Sodium acetate (with a final concentration of 0.3 M) was added to the salting-out supernatant obtained in step 3 and sufficiently stirred and evenly mixed; upon complete dissolution of sodium acetate, absolute ethanol with a volume 2.5 times that of the supernatant was slowly added to the supernatant for 30 min of precipitation. The mixture was centrifuged at 9000 rpm for 10 min, and the supernatant was discarded; the precipitate was rinsed once with 75% ethanol and then centrifuged at 9000 rpm for 10 min, and then the supernatant was discarded to obtain a wet product precipitate.
Step 5, obtaining of a product via dehydration and drying:
   20 mL of absolute ethanol was added to the rinsed precipitate for dehydration. After the precipitate was fully dispersed, the mixture was centrifuged at 9000 rpm for 10 min, and the supernatant was discarded (the operations were repeated 3 times). The resulting precipitate was transferred to a disposable culture dish and dried in vacuum at 40°C until the weight of the product no longer changed significantly (the weight variation of the product was less than 0.001 g). Upon completion of the drying, the PDRN product was obtained.

### Test example 15

This test example provides a preparation method of polydeoxyribonucleotide (PDRN) derived from *Anabaena variabilis* ATCC 29413 (purchased from the American Type Culture Collection under the deposit number ATCC 29413), specifically comprising the following steps:
Step 1, preparation of a culture solution via culture of *Anabaena variabilis*:
   The *Anabaena variabilis* strain was inoculated into a 8-fold diluted AA liquid medium (purchased from Beijing Solarbio Science & Technology Co., Ltd., Cat. No. LA8520), followed by static culture at 30°C under an illumination intensity of 30 µE/(m² s) for 5 days to obtain a culture solution of *Anabaena variabilis*.
Step 2, harvesting and resuspension of algal pellets and preparation of an algal lysate (300 mL of culture solution was taken as an example):
   300 mL of culture solution (culture solution of *Anabaena variabilis*) was centrifuged at 9000 rpm for 5 min, and the supernatant was discarded. The algal pellets were rinsed once with 300 mL of TE buffer and centrifuged again at 9000 rpm for 5 min. 20 mL of TE buffer was added to resuspend the algal pellets evenly. Lysozyme was added to the TE-resuspended algal pellets to a final concentration of 0.2 mg/mL, followed by constant-temperature incubation at 37°C for 2 h. The mixture was subjected to high-pressure homogenization using a high-pressure homogenizer at a constant pressure of 1000 bar for 3 cycles to prepare the algal lysate.
Step 3, preparation of a salting-out supernatant via salting-out and centrifugation:
   NaCl (with a final concentration of 2.5 M) and SDS (sodium dodecyl sulfate, with a final concentration of 1%) were added to the obtained algal lysate, followed by incubation at 60°C for 1.5 h. The supernatant was collected via centrifugation to prepare the salting-out supernatant.
Step 4, preparation of a crude product via ethanol precipitation:
   Sodium acetate (with a final concentration of 0.3 M) was added to the salting-out supernatant obtained in step 3 and sufficiently stirred and evenly mixed; upon complete dissolution of sodium acetate, absolute ethanol with a volume 2.5 times that of the supernatant was slowly added to the supernatant for 30 min of precipitation. The mixture was centrifuged at 9000 rpm for 10 min, and the supernatant was discarded; the precipitate was rinsed once with 75% ethanol and then centrifuged at 9000 rpm for 10 min, and then the supernatant was discarded to obtain a wet product precipitate.
Step 5, obtaining of a product via dehydration and drying:
   20 mL of absolute ethanol was added to the rinsed precipitate for dehydration. After the precipitate was fully dispersed, the mixture was centrifuged at 9000 rpm for 10 min, and the supernatant was discarded (the operations were repeated 3 times). The resulting precipitate was transferred to a disposable culture dish and dried in vacuum at 40°C until the weight of the product no longer changed significantly (the weight variation of the product was less than 0.001 g). Upon completion of the drying, the PDRN product was obtained.

### Test example 16

This test example provides a preparation method of polydeoxyribonucleotide (PDRN) derived from guinea pigs (*Cavia porcellus* (conventional grade, purchased from Liaoning Changsheng Biotechnology Co., Ltd.)), comprising the following steps:
Step 1, guinea pig raising
   Three-week-old conventional-grade guinea pigs purchased from Liaoning Changsheng Biotechnology Co., Ltd. were normally raised for 3 months.
Step 2, collection and digestion of guinea pig liver tissues and preparation of a tissue lysate (1 g of liver tissue was taken as an example):
   The raised guinea pigs were sacrificed by cervical dislocation. The guinea pig samples were dissected by routine methods to collect liver tissues which were frozen in liquid nitrogen for 10 min. The frozen liver tissues were taken out and ground into fine powder in liquid nitrogen.1 g of tissue powder was weighed and mixed with 20 mL of TE buffer for uniform resuspension. Proteinase K (purchased from Beijing Solarbio Science & Technology Co., Ltd., Cat. No. P9460) was added to the TE resuspension to a final concentration of 2 mg/mL, followed by constant-temperature incubation at 56°C for 2 h to prepare the tissue lysate.
Step 3, preparation of a salting-out supernatant via salting-out and centrifugation:
   NaCl (with a final concentration of 2.5 M) and SDS (sodium dodecyl sulfate, with a final concentration of 1%) were added to the obtained tissue lysate, followed by incubation at 60°C for 1.5 h. The supernatant was collected via centrifugation to prepare the salting-out supernatant.
Step 4, preparation of a crude product via ethanol precipitation:
   Sodium acetate (with a final concentration of 0.3 M) was added to the salting-out supernatant obtained in step 3 and sufficiently stirred and evenly mixed; upon complete dissolution of sodium acetate, absolute ethanol with a volume 2.5 times that of the supernatant was slowly added to the supernatant for 30 min of precipitation. The mixture was centrifuged at 9000 rpm for 10 min, and the supernatant was discarded; the precipitate was rinsed once with 75% ethanol and then centrifuged at 9000 rpm for 10 min, and then the supernatant was discarded to obtain a wet product precipitate.
Step 5, obtaining of a product via dehydration and drying:
   20 mL of absolute ethanol was added to the rinsed precipitate for dehydration. After the precipitate was fully dispersed, the mixture was centrifuged at 9000 rpm for 10 min, and the supernatant was discarded (the operations were repeated 3 times). The resulting precipitate was transferred to a disposable culture dish and dried in vacuum at 40°C until the weight of the product no longer changed significantly (the weight variation of the product was less than 0.001 g). Upon completion of the drying, the PDRN product was obtained.

In Comparative example 1, the PDRN product was prepared by using the method disclosed in CN107287186A of the background.

In Comparative example 2, the PDRN product was prepared by using the method disclosed in CN112315836A of the background.

In Comparative example 3, the PDRN product was prepared by using the method disclosed in CN115074357A of the background.

The PDRN products obtained in test examples 1-16 and Comparative examples 1-3 were subjected to the detection described below.

### Purity and nucleic acid content determination:

An appropriate amount of dried product described above was accurately weighed and redissolved in water at the concentration of 5 mg/mL. Following incubation at 40°C for 30 min, the solution was mixed by gentle inversion (the operations were repeated 2-3 times until complete dissolution of the product). The resultant solution was used for the determination of polynucleotide purity. After the aforementioned dried product was dissolved in water, the absorbance values of the redissolved sample at 260 nm and 280 nm were measured using an ultra-micro ultraviolet spectrophotometer. The purity and nucleic acid content of PDRN in the product were determined according to the results output by the instrument. The calculation formulas for purity are as follows: Product purity = OD₂₆₀/OD₂₈₀; Nucleic acid content = measured sample concentration (ng/µL)/(5 mg/mL)/1000 × 100%.

### Protein determination:

In this example, the protein content in the product was determined via a BCA protein quantification kit.

First, a BCA working solution (both BCA Reagent A and BCA Reagent B were included in the kit.) was prepared by thoroughly mixing 50 volumes of BCA Reagent A and 1 volume of BCA Reagent B (50:1).

A microplate was taken, and reagents were added according to the data shown in Table 1. After shaking and mixing thoroughly, the microplate was incubated at 37°C for 30 min. The absorbance at A562 nm was measured with a microplate reader. A standard curve was plotted by using the protein content (µg) as the abscissa and the absorbance as the ordinate.

**Table 1**

| | | | | | | | | |
|---|---|---|---|---|---|---|---|---|
| Pore number | 0 | 1 | 2 | 3 | 4 | 5 | 6 | 7 |
| Standard protein * (µL) | 0 | 1 | 2 | 4 | 8 | 12 | 16 | 20 |
| Deionized water (µL) | 20 | 19 | 18 | 16 | 12 | 8 | 4 | 0 |
| BCA working solution (µL) | 200 | 200 | 200 | 200 | 200 | 200 | 200 | 200 |
| Corresponding protein content (g/L) | 0 | 0.05 | 0.1 | 0.2 | 0.4 | 0.6 | 0.8 | 1 |

| | | | | | | | | |
|---|---|---|---|---|---|---|---|---|
| *: The standard protein is contained in the kit. | | | | | | | | |

Sample determination: 100 mg of PDRN sample was accurately weighed and dissolved in 200 mL of deionized water to prepare a 0.5 g/L sample solution. 200 µL of BCA working solution was added to 20 µL of the prepared solution. After vortex mixing, the mixture was incubated at 37°C for 30 min. The absorbance at A562 nm was measured using the microplate reader, and the absorbance of the blank without the standard protein was used as the blank control. The protein content in the test sample was calculated by substituting the measured absorbance value into the standard curve.$Protein \left(%\right) of product = \left(protein content in test sample/0.5\right) \times 100 % .$

### Molecular weight determination:

In this example, the molecular weight of the product was determined by capillary electrophoresis using an automatic nucleic acid analyzer.

The instruments and equipment used included an automatic nucleic acid analyzer (model Qsep100), electronic balance, vortex mixer, ultra-micro ultraviolet spectrophotometer, and others.

Test sample solution: 0.01 g of sample was weighed, dissolved in water and diluted to prepare a solution containing approximately 5 mg per 1 mL (0.5%). The sample solution was further diluted 100 times to a final sample concentration of 50 ng/µL.

Instrument loading test: the power supply of the instrument and the switch of the air compressor were turned on, and the software icon was double-clicked; a new project was created by selecting New Project; the "Connect" button was clicked to establish system connection; the buffer solution and Marker were configured; the "Change buffer" button was clicked, and the Marker and buffer solution were loaded; the sample plate was installed; the "Change Sample" button was clicked to open the transparent door cover, and samples were placed inside (sample volume ≥ 10 µL); holes were punched on the new cartridge holder; the cartridge holder door was opened, the cartridge holder was placed inside, and then the cartridge holder door was tightly closed; the "Latch" button was clicked, followed by cartridge holder calibration; blank areas were clicked, and the following items were selected in sequence: (1) sample position; (2) test protocol, injection time, test runs; (3) result file name; (4) calculation.

Data processing: After the program run was completed, partial short fragments with low content and excessively long fragments were manually excluded. The weight-average molecular weight was calculated (the molecular weight of the product was automatically computed by the automatic nucleic acid analyzer).

### Base and nucleotide determination:

In this example, the contents of bases and nucleotides in the product were determined by using a perchloric acid hydrolysis method.

5 mg of test sample was separately weighed and transferred into 6 tubes, with three tubes in each group. 300 µL of 70% perchloric acid was added, while an equal volume of water was added to the control group. Subsequently, the tubes were sealed to form ampoules. The mixture was heated in boiling water at 100°C for 1 h. After cooling, the ampoules were opened, and all contents were completely transferred into a 25 mL volumetric flask. The pH value was adjusted to 5.3 with 6 M potassium hydroxide, and the solution was brought to volume with water. The solution was filtered through a 0.22 µm MCE membrane filter prior to measurement.

Preparation of a standard solution: adenine and uracil were separately dissolved with a 0.1 M KOH solution, while cytidine nucleotide and uridine nucleotide were separately dissolved with ultrapure water to reach a final concentration of 1 mg/mL. Serial dilutions were performed with ultrapure water to obtain concentrations of 0.1 µg/mL, 1 µg/mL, 10 µg/mL, 0.1 mg/mL and 1 mg/mL, respectively. The solution was filtered through a 0.22 µm aqueous MCE membrane filter, followed by liquid phase detection.

Analysis of bases and nucleotide: An Agilent ZORBAX EC-C18 chromatographic column (4 µm, 4.6 mm × 150 mm) was used. Mobile phase A was 5 mM ammonium acetate solution; mobile phase B was methanol. Gradient elution program: 0-10 min, 0%-5% methanol; 10-30 min, 5%-20% methanol. The flow rate was set to 0.45 mL/min. The temperature of the column was maintained at 25°C. The detection wavelength of the DAD detector was set to 254 nm, and an injection volume of 5 µL was used for chromatogram acquisition and quantitative analysis. GC% was calculated via high-performance liquid chromatography integration.

### Appearance and solubility assay:

In this example, the appearance of the product was observed and recorded, and the solubility of the product was relevantly assayed. The method is as follows:

### Observation of appearance:

By visual observation, the test sample should be white or off-white granules or powder in appearance.

### Solubility test:

1 g of test sample was weighed and added to 1 L of pure water to dilute the sample to a final volume of 1 L. The mixture was gently mixed. After standing for 30 min, the solubility was observed. If the resulting solution was clear and transparent without impurities or turbidity, the test sample passed the solubility test; otherwise, it failed the solubility test.

The test results of examples and comparative examples described above and their comparison are shown in Table 2.

**Table 2**

| Sample | Appearance | Solubility* | Puri ty | Nucleic acid content | Protein content | Molecular weight | GC% |
|---|---|---|---|---|---|---|---|
| Test example 1 | White powder | Completely dissolved | 1.95 | 99.50% | 0.19% | 249.5 kDa | 40.91% |
| Test example 2 | White powder | Completely dissolved | 1.84 | 97.10% | 0.21% | 430.3 kDa | 40.94% |
| Test example 3 | White powder | Completely dissolved | 1.82 | 96.30% | 0.43% | 186.6 kDa | 40.98% |
| Test example 4 | White powder | Completely dissolved | 1.93 | 98.70% | 0.23% | 373.8 kDa | 40.83% |
| Test example 5 | White powder | Completely dissolved | 1.87 | 99.10% | 0.37% | 493.9 kDa | 41.06% |
| Test example 6 | White powder | Completely dissolved | 1.84 | 96.01% | 0.41% | 253.7 kDa | 40.93% |
| Test example 7 | White powder | Completely dissolved | 1.83 | 95.10% | 0.35% | 237.2 kDa | 46.72% |
| Test example 8 | White powder | Completely dissolved | 1.92 | 98.01% | 0.39% | 223.4 kDa | 41.49% |
| Test example 9 | White powder | Completely dissolved | 1.93 | 99.31% | 0.19% | 249.1 kDa | 41.49% |
| Test example 10 | White powder | Completely dissolved | 1.85 | 99.10% | 0.24% | 237.6 kDa | 41.33% |
| Test example 11 | White powder | Completely dissolved | 1.84 | 98.73% | 0.21% | 241.8 kDa | 41.41% |
| Test example 12 | White powder | Completely dissolved | 1.90 | 99.28% | 0.25% | 231.9 kDa | 41.25% |
| Test example 13 | White powder | Completely dissolved | 1.88 | 99.01% | 0.18% | 271.7 kDa | 41.09% |
| Test example 14 | White powder | Completely dissolved | 1.93 | 98.10% | 0.45% | 288.2 kDa | 41.16% |
| Test example 15 | White powder | Completely dissolved | 1.97 | 97.76% | 0.33% | 437.7 kDa | 41.50% |
| Test example 16 | White powder | Completely dissolved | 1.93 | 98.10% | 0.45% | 513.9 kDa | 39.89% |
| Comparative example 1 | Pale yellow powder | A small amount of insoluble matter remained | 1.73 | 90.10% | 1.81% | 255.6 kDa | 43.24% |
| Comparative example 2 | White powder | Completely dissolved | 1.85 | 98.60% | 0.79% | 215.9 kDa | 43.41% |
| Comparative example 3 | White powder | Completely dissolved | 1.84 | 98.30% | 0.31% | 239.8 kDa | 43.32% |

| | | | | | | | |
|---|---|---|---|---|---|---|---|
| *: 1 g of product was dissolved in 1 L of water within 30 min. | | | | | | | |

It can be seen from Table 2 that the PDRN product prepared by the method described in test examples possesses high purity and high nucleic acid content, as well as low protein content. The GC content of the PDRN product ranges from 39.5% to 41.5%, which is very close to the human GC content of 40% with a homology of more than 98%. In addition, the product exhibits high nucleic acid content, high molecular weight and high yield, making it suitable for industrial production.

### Example 3 Efficacy assay

The PDRN products prepared in Test examples 1 and/or 2 and/or 3 and/or 4 and/or 5 and/or 6 and/or 7 and/or 8 and/or 9 and/or 10 and/or 11 and/or 12 and/or 13 and/or 14 and/or 15 and/or 16 in Example 2 and Comparative examples 1-3 were used as samples for efficacy assay.

### Cytotoxicity assay:

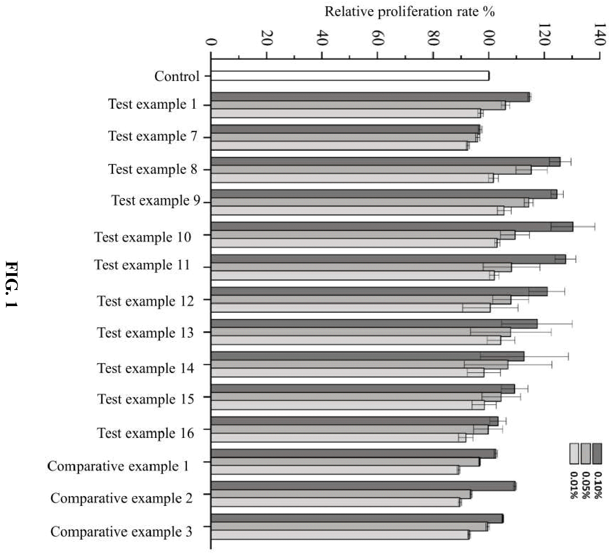

Specifically, the above samples in Test examples 1 and 7-16 and Comparative examples 1-3 were subjected to cytotoxicity assay. Three concentrations of 0.01%, 0.05% and 0.1% were tested separately for each sample. Information of the tested samples is shown in Table 3. HS27 cells were used as experimental subjects. The culture system was DMEM medium (supplemented with 10% fetal bovine serum), and the cells were cultured under culture conditions of 37°C and 5% CO₂. After 24 h of cell culture treatment, the original medium was discarded, and serum-free medium containing the samples was added for another 24 h of incubation. The concentrations of the samples are listed in Table 3, and water was used as the control group. The CCK-8 method was used to determine the relative cell proliferation rate. The relative cell proliferation rate data are presented in Table 4. FIG. 1 was plotted based on the data in Table 4.

**Table 3**

| Sample name | Sample concentration% |
|---|---|
| Test example 1 | 0.01, 0.05, 0.1 |
| Test example 7 | 0.01, 0.05, 0.1 |
| Test example 8 | 0.01, 0.05, 0.1 |
| Test example 9 | 0.01, 0.05, 0.1 |
| Test example 10 | 0.01, 0.05, 0.1 |
| Test example 11 | 0.01, 0.05, 0.1 |
| Test example 12 | 0.01, 0.05, 0.1 |
| Test example 13 | 0.01, 0.05, 0.1 |
| Test example 14 | 0.01, 0.05, 0.1 |
| Test example 15 | 0.01, 0.05, 0.1 |
| Test example 16 | 0.01, 0.05, 0.1 |
| Comparative example 1 | 0.01, 0.05, 0.1 |
| Comparative example 2 | 0.01, 0.05, 0.1 |
| Comparative example 3 | 0.01, 0.05, 0.1 |

**Table 4**

| Sample name | Sample concentration | Relative proliferation rate % | |
|---|---|---|---|
| | | Mean | Variance |
| Control | - | 100.11 | 0.19 |
| Test example 1 | 0.10% | 114.8 | 0.63 |
| | 0.05% | 106.1 | 1.48 |
| | 0.01% | 97.15 | 0.92 |
| Test example 7 | 0.10% | 96.94 | 0.67 |
| | 0.05% | 96.11 | 0.8 |
| | 0.01% | 92.51 | 0.56 |
| Test example 8 | 0.10% | 125.67 | 3.92 |
| | 0.05% | 115.42 | 5.56 |
| | 0.01% | 101.78 | 1.78 |
| Test example 9 | 0.10% | 124.56 | 2.21 |
| | 0.05% | 114.56 | 1.63 |
| | 0.01% | 105.63 | 2.45 |
| Test example 10 | 0.10% | 130.33 | 7.94 |
| | 0.05% | 109.53 | 5.20 |
| | 0.01% | 103.13 | 0.96 |
| Test example 11 | 0.10% | 127.63 | 3.75 |
| | 0.05% | 108.21 | 10.21 |
| | 0.01% | 102.05 | 1.79 |
| Test example 12 | 0.10% | 121.04 | 6.38 |
| | 0.05% | 107.98 | 6.49 |
| | 0.01% | 100.57 | 10.02 |
| Test example 13 | 0.10% | 117.40 | 12.67 |
| | 0.05% | 107.94 | 14.53 |
| | 0.01% | 104.48 | 5.05 |
| Test example 14 | 0.10% | 112.86 | 15.83 |
| | 0.05% | 106.96 | 15.83 |
| | 0.01% | 98.27 | 6.05 |
| Test example 15 | 0.10% | 109.37 | 4.78 |
| | 0.05% | 104.56 | 6.93 |
| | 0.01% | 98.45 | 4.34 |
| Test example 16 | 0.10% | 103.45 | 3.01 |
| | 0.05% | 99.87 | 5.26 |
| | 0.01% | 91.78 | 2.67 |
| Comparative example 1 | 0.10% | 102.64 | 0.56 |
| | 0.05% | 96.78 | 0.3 |
| | 0.01% | 89.23 | 0.5 |
| Comparative example 2 | 0.10% | 109.48 | 0.51 |
| | 0.05% | 93.63 | 0.44 |
| | 0.01% | 89.57 | 0.59 |
| Comparative example 3 | 0.10% | 105.16 | 0.31 |
| | 0.05% | 99.64 | 0.64 |
| | 0.01% | 92.99 | 0.51 |

The cytotoxicity assay results shown in Table 4 and FIG. 1 indicate that the products prepared in Test examples 1 and 8-15 do not possess cytotoxicity (with a cell proliferation rate of > 80%), the overall cell proliferation-promoting effects of PDRN derived from *Lactobacillus sakei*, *Eubacterium rectale*, *Bacillus pumilus*, *Komagataella sp.* and *Anabaena variabilis* are better than those in Test example 7 and Comparative examples 1-3, and superior to that of PDRN derived from guinea pigs.

### UV repair efficacy assay:

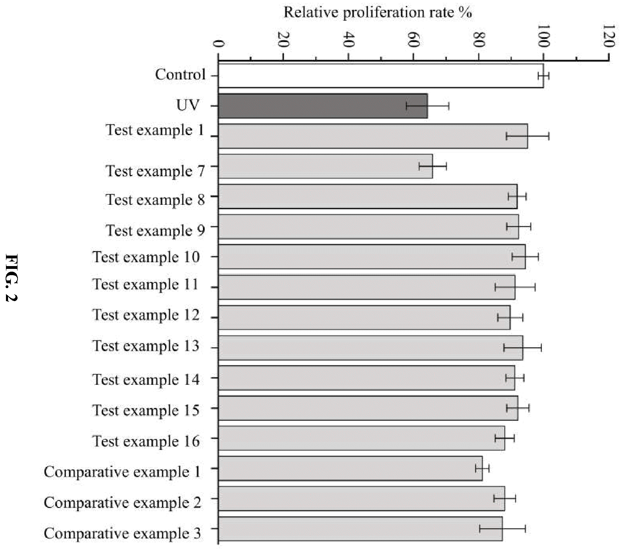

The samples (sample concentrations were all 0.01%) prepared in Test examples 1 and 7-16 and Comparative examples 1-3 were subjected to UV repair efficacy assay. Specifically, HS27 cells were used as experimental subjects. The culture system was DMEM medium (supplemented with 10% fetal bovine serum), and the cells were cultured under culture conditions of 37°C and 5% CO₂. After the cells were irradiated with ultraviolet light (2000 µW/cm² for 80 min), the original medium was discarded, and a serum-free medium containing the samples was added, followed by further incubation for 24 h. The relative cell proliferation rate was determined by using a CCK-8 method. The control group received no UV irradiation. The UV group was only supplemented with an equal volume of serum-free medium without samples after UV irradiation. Relative proliferation rate data is shown in Table 5. FIG. 2 was plotted according to the data in Table 5.

**Table 5**

| Sample name | Relative cell proliferation rate % | Variance |
|---|---|---|
| Control | 100.00 | 1.63 |
| UV | 64.32 | 6.50 |
| Test example 1 | 95.08 | 6.51 |
| Test example 7 | 65.93 | 4.15 |
| Test example 8 | 91.89 | 2.74 |
| Test example 9 | 92.34 | 3.71 |
| Test example 10 | 94.38 | 4.02 |
| Test example 11 | 91.23 | 6.15 |
| Test example 12 | 89.76 | 3.86 |
| Test example 13 | 93.57 | 5.76 |
| Test example 14 | 91.18 | 2.76 |
| Test example 15 | 92.08 | 3.45 |
| Test example 16 | 88.01 | 2.92 |
| Comparative example 1 | 81.17 | 2.07 |
| Comparative example 2 | 88.05 | 3.33 |
| Comparative example 3 | 87.37 | 7.04 |

The UV repair efficacy assay results shown in Table 5 and FIG. 2 indicate that the products prepared in Test examples 1 and 8-16 have better UV repair effects than those of the PDRN products prepared in Test example 7 and Comparative examples 1-3. Furthermore, the UV repair effects of the PDRN derived from *Lactobacillus sakei*, *Eubacterium rectale*, *Bacillus pumilus*, *Komagataella sp.* and *Anabaena variabilis* are superior to that of PDRN derived from guinea pigs.

### Trauma repair efficacy assay

The above samples from Test examples 1 and 7-16 and Comparative examples 1-3 (all tested at a sample concentration of 0.01%) were further subjected to trauma repair efficacy assay.

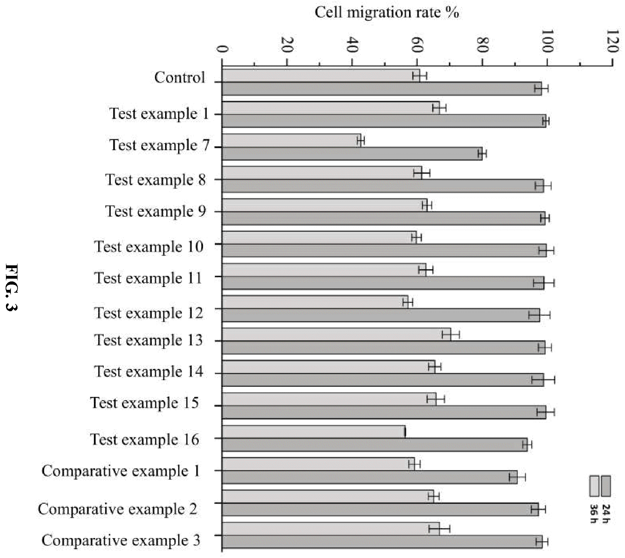

Specifically, HS27 cells were used as experimental subjects. The culture system was DMEM medium (supplemented with 10% fetal bovine serum), and the cells were cultured under culture conditions of 37°C and 5% CO₂. Sample treatment process: ibidi inserts were placed in a 24-well culture plate. After cells were cultured for 24 h, the ibidi inserts were removed. Scratches were photographed, then the original medium was discarded, and a serum-free medium containing the samples was added for further incubation for 24 h, 36 h and 48 h (with observation and photography at each time point). The control group was only supplemented with an equal volume of serum-free medium without samples. The area of the scratch region in the photographs was analyzed using ImageJ software. Cell migration rate (%) = (1 - scratch area after sample treatment/initial scratch area) × 100. The cell migration rate data is shown in Table 6. FIG. 3 was plotted according to the data in Table 6.

**Table 6**

| Sample name | Cell migration rate% 24 h | Variance | Cell migration rate% 36 h | Variance |
|---|---|---|---|---|
| Control | 60.80 | 2.11 | 98.13 | 2.10 |
| Test example 1 | 66.85 | 2.04 | 99.51 | 0.99 |
| Test example 7 | 42.67 | 1.04 | 79.98 | 1.28 |
| Test example 8 | 61.45 | 2.46 | 98.75 | 2.48 |
| Test example 9 | 63.08 | 1.43 | 99.24 | 1.37 |
| Test example 10 | 59.83 | 1.43 | 99.67 | 2.34 |
| Test example 11 | 62.71 | 2.19 | 98.91 | 3.19 |
| Test example 12 | 57.19 | 1.46 | 97.57 | 3.27 |
| Test example 13 | 70.35 | 2.59 | 99.26 | 2.07 |
| Test example 14 | 65.43 | 1.88 | 98.74 | 3.52 |
| Test example 15 | 65.78 | 2.63 | 99.47 | 2.67 |
| Test example 16 | 56.35 | 0.18 | 93.79 | 1.39 |
| Comparative example 1 | 59.23 | 1.72 | 90.76 | 2.44 |
| Comparative example 2 | 65.10 | 1.64 | 97.22 | 2.18 |
| Comparative example 3 | 66.86 | 3.19 | 98.32 | 1.88 |

The results in Table 6 and FIG. 3 indicate that after treatment with PDRNs of different sources for 24 h, the PDRNs prepared in Test examples 1 and 8-15 of the present invention exhibit significantly superior effects in promoting cell migration compared with PDRNs prepared by other processes and from other sources. This indicates that PDRNs derived from *Lactobacillus sakei*, *Eubacterium rectale*, *Bacillus pumilus*, *Komagataella sp.* and *Anabaena variabilis* and prepared via the process disclosed herein possess excellent trauma repair efficacy.

### Soothing efficacy assay

The samples (with concentrations of 0.01%) prepared in Test examples 1 and 7-16 and Comparative examples 1-3 were subjected to soothing efficacy assay.

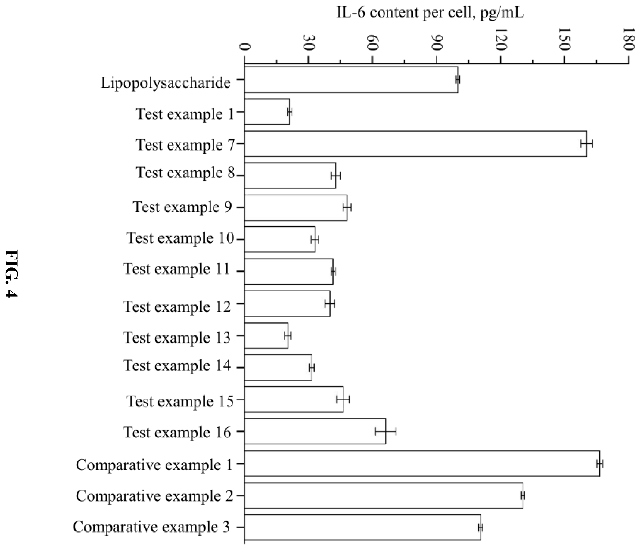

Specifically, Raw264.7 cells were used as experimental subjects. The culture system was RPMI1640 medium (supplemented with 10% fetal bovine serum), and the cells were cultured under culture conditions of 37°C and 5% CO₂. Sample treatment process: after cell culture for 24 h, the original medium was discarded, and a serum-free medium supplemented with the test samples was added for another 24 h incubation (lipopolysaccharide (LPS) was used as model control). The CCK-8 method was used to measure the relative cell proliferation rate. Cell supernatants were collected by centrifugation, and inflammatory factors were detected in accordance with the instructions of the ELISA kit. The relevant data is shown in Table 7. FIG. 4 was plotted according to the data in Table 7.

**Table 7**

| Sample name | IL-6 content per cell pg/mL | Variance |
|---|---|---|
| Lipopolysaccharide | 100.00 | 0.88 |
| Test example 1 | 21.26 | 0.99 |
| Test example 7 | 160.40 | 2.69 |
| Test example 8 | 42.93 | 2.19 |
| Test example 9 | 48.25 | 1.94 |
| Test example 10 | 32.94 | 1.65 |
| Test example 11 | 41.71 | 0.91 |
| Test example 12 | 40.18 | 2.16 |
| Test example 13 | 20.33 | 1.47 |
| Test example 14 | 31.47 | 1.06 |
| Test example 15 | 46.38 | 2.86 |
| Test example 16 | 66.25 | 4.83 |
| Comparative example 1 | 166.53 | 1.29 |
| Comparative example 2 | 130.37 | 0.80 |
| Comparative example 3 | 110.67 | 0.87 |

The results in Table 7 and FIG. 4 indicate that after the cell line modeled with LPS is treated with the products prepared in Test examples 1 and 8-15 of the present invention, the level of inflammatory factor IL-6 is markedly reduced. This demonstrates that PDRNs derived from *Lactobacillus sakei*, *Eubacterium rectale*, *Bacillus pumilus*, *Komagataella sp.* and *Anabaena variabilis* and prepared by the process disclosed herein exert excellent soothing efficacy.

### Collagen-promoting efficacy assay

The samples (all at a sample concentration of 0.01%) prepared in Test examples 1 and 7-16 and Comparative examples 1-3 were further subjected to collagen-promoting efficacy assay.

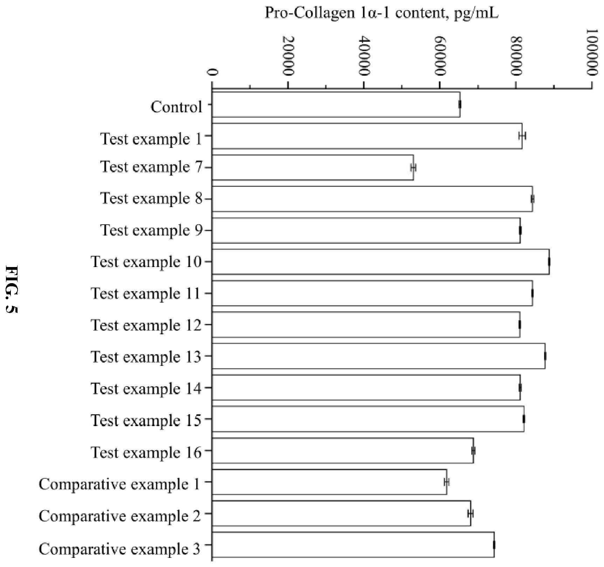

Specifically, HS27 cells were used as experimental subjects. The culture system was DMEM medium (supplemented with 10% fetal bovine serum), and the cells were cultured under culture conditions of 37°C and 5% CO₂. Sample treatment process: after cell culture for 24 h, the original medium was discarded, and a serum-free medium supplemented with the samples was added for another 24 h incubation. The cell supernatant was collected. An equal volume of serum-free medium was added in control group without addition of samples. Pro-Collagen Iα-1 (Pro-collagen Iα-1) content was determined with reference to Pro-Collagen Iα-1 ELISA kit. The relevant data is shown in Table 8. FIG. 5 was plotted according to the data in Table 8.

**Table 8**

| Sample name | Pro-collagen I α-1 content pg/mL | Variance |
|---|---|---|
| Control | 65196.50 | 249.50 |
| Test example 1 | 81614.83 | 873.58 |
| Test example 7 | 53030.33 | 615.67 |
| Test example 8 | 84321.87 | 321.87 |
| Test example 9 | 81098.54 | 210.76 |
| Test example 10 | 88763.12 | 160.38 |
| Test example 11 | 84321.89 | 182.53 |
| Test example 12 | 80987.65 | 204.29 |
| Test example 13 | 87654.98 | 170.98 |
| Test example 14 | 81109.78 | 292.47 |
| Test example 15 | 82109.65 | 210.76 |
| Test example 16 | 68765.32 | 432.98 |
| Comparative example 1 | 61816.50 | 588.50 |
| Comparative example 2 | 68020.33 | 659.53 |
| Comparative example 3 | 74247.33 | 175.28 |

The results in Table 8 and FIG. 5 indicate that after the products prepared in Test examples 1 and 8-15 are treated for 24 h, the Pro-collagen Iα-1 contents in the cells significantly increase. It is demonstrated that PDRNs derived from *Lactobacillus sakei*, *Eubacterium rectale*, *Bacillus pumilus*, *Komagataella sp.* and *Anabaena variabilis* and prepared by the process disclosed herein can significantly boost collagen synthesis in cells, with better efficacy than that of PDRN derived from guinea pigs.

It has been verified that the PDRN products derived from *Lactobacillus sakei*, *Eubacterium rectale*, *Bacillus pumilus*, *Komagataella sp.* and *Anabaena variabilis* and prepared by the method described in the present invention exhibit no cytotoxicity, and possess UV repair efficacy, trauma repair efficacy and collagen-promoting efficacy.

The above descriptions are embodiments of the present invention, but are not intended to limit the present invention. For those skilled in the art, various variations and changes can be made to the present invention. Any amendments, equivalent replacements, improvements and the like made within the spirit and principle of the present invention shall be included within the scope of the appended claims of the present invention.

## Claims

1. A polydeoxyribonucleotide/polyribonucleotide (PDRN), wherein the PDRN has the GC content of 40.5%-42%.

2. The PDRN according to claim 1, wherein the weight-average molecular weight of the PDRN is 100 kDa-500 kDa.

3. A preparation method of PDRN, the method comprising the following steps:
Step 1, collecting materials, and then lysing the materials to prepare a lysate;
Step 2, subjecting the lysate to salting out, so as to prepare a salting-out supernatant;
Step 3, adding an organic solvent to the salting-out supernatant for precipitation to prepare a crude product; and
Step 4, drying the crude product to obtain a product;
optionally, the materials comprising microorganisms, animals and/or algae;
more optionally, the animals comprising one or more selected from *Cavia porcellus*, *Caenorhabditis japonica*, *Drosophila ananassae*, *Branchiostoma floridae*, *Oryzias latipes* and *Gallus gallus*, and more optionally the animals comprising *Cavia porcellus*;
optionally, the algae comprising freshwater algae, more optionally, the algae comprising *Anabaena variabilis*;
more optionally, the microorganisms comprising one or more selected from *Lactobacillus, Bacillus, Eubacterium* and yeasts, and more optionally the microorganisms comprising one or more selected from *Lactobacillus sakei*, *Eubacterium rectale*, *Bacillus pumilus*, *Komagataella sp.* and *Lactobacillus reuteri.*

4. The method according to claim 3, the method comprising the following steps:
Step 1, collecting materials, resuspending the materials, and then adding an enzyme for lysis to prepare a lysate;
Step 2, adding a salt and a protein denaturant to the lysate for salting out to prepare a salting-out supernatant;
Step 3, adding an organic solvent to the salting-out supernatant for precipitation to prepare a crude product; and
Step 4, drying the crude product to obtain a product;
optionally, the enzyme comprising one or more selected from Labiase derived from *Streptomyces fulvissimus*, mutanolysin, lysostaphin, achromopeptidase, papain, Proteinase K and snailase; optionally, the enzyme is added to a final concentration of 0.2-2 mg/mL;
optionally, the salt comprising one or more selected from sodium chloride, potassium acetate, sodium acetate, ammonium sulfate and sodium sulfate; optionally, the final concentration of the salt added being 1-5 M;
optionally, the protein denaturant comprising one or more selected from guanidine hydrochloride, phenol and a surfactant;
optionally, the organic solvent comprising one or more selected from ethanol, methanol and isopropanol.

5. A composition, the composition comprising the PDRN according to claim 1 or 2 or the PDRN prepared by the method according to claim 3 or 4.

6. Use, the use at least comprising any one or more of A1)-A7):
A1) use of *Lactobacillus sakei* in the preparation of a PDRN product;
A2) use of *Eubacterium rectale* in the preparation of a PDRN product;
A3) use of *Bacillus pumilus* in the preparation of a PDRN product;
A4) use of *Komagataella sp.* in the preparation of a PDRN product;
A5) use of *Lactobacillus reuteri* in the preparation of a PDRN product;
A6) use of *Cavia porcellus* in the preparation of a PDRN product; and
A7) use of *Anabaena variabilis* in the preparation of a PDRN product;

7. *Bacillus pumilus*, wherein the *Bacillus pumilus* is *Bacillus pumilus* Bp-1 which is deposited at the China Center for Type Culture Collection under the deposit number CCTCC NO: M 2025198.

8. *Bacillus pumilus*, wherein the *Bacillus pumilus* is *Bacillus pumilus* Bp-2 which is deposited at the China Center for Type Culture Collection under the deposit number CCTCC NO: M 2025199.

9. *Komagataella sp.*, wherein the *Komagataella sp.* is *Komagataella sp.* GS115 which is deposited at the China Center for Type Culture Collection under the deposit number CCTCC NO: M 2025200.

10. *Lactobacillus sakei*, wherein the *Lactobacillus sakei* is *Lactobacillus sake* LS-01 which is deposited at the China Center for Type Culture Collection under the deposit number CCTCC NO: M 20232285.

11. Use of the PDRN according to claim 1 or 2 or the PDRN obtained by the method according to claim 3 or 4 or the composition according to claim 5 or the *Bacillus pumilus* according to claim 7 or the *Bacillus pumilus* according to claim 8 or the *Komagataella sp.* according to claim 9 or the *Lactobacillus sakei* according to claim 10 or the *Anabaena variabilis* according to claim 3 in the preparation of a product for facilitating cell proliferation and/or cell migration and/or facilitating collagen generation.

12. Use of the PDRN according to claim 1 or 2 or the PDRN obtained by the method according to claim 3 or 4 or the composition according to claim 5 or the *Bacillus pumilus* according to claim 7 or the *Bacillus pumilus* according to claim 8 or the *Komagataella sp.* according to claim 9 or the *Lactobacillus sakei* according to claim 10 or the *Anabaena variabilis* according to claim 3 in the preparation of a product for exerting anti-inflammatory and soothing effects and/or repairing damaged skin barriers.
